(19) Europäisches Patentamt
European Patent Office
Office européen des brevets

(11) EP 3 991 750 A1

(12) EUROPEAN PATENT APPLICATION
published in accordance with Art. 153(4) EPC

(43) Date of publication:
**04.05.2022 Bulletin 2022/18**

(21) Application number: **20833680.0**

(22) Date of filing: **10.01.2020**

(51) International Patent Classification (IPC):
***A61K 45/00*** (2006.01) ***A61K 9/12*** (2006.01)
***A61K 9/14*** (2006.01) ***A61K 47/26*** (2006.01)

(52) Cooperative Patent Classification (CPC):
**A23L 5/00; A61K 9/12; A61K 9/14; A61K 31/192; A61K 31/277; A61K 31/506; A61K 31/517; A61K 31/5377; A61K 38/13; A61K 45/00; A61K 47/26; B01J 2/04**

(86) International application number:
**PCT/JP2020/000711**

(87) International publication number:
**WO 2020/261619 (30.12.2020 Gazette 2020/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.06.2019 JP 2019118289**

(71) Applicants:
- **Ricoh Company, Ltd.**
  **Tokyo 143-8555 (JP)**
- **Shinshu University**
  **Matsumoto-shi, Nagano 390-8621 (JP)**

(72) Inventors:
- **SATO, Takashi**
  **Kamiina-gun, Nagano 399-4598 (JP)**
- **SHIMOSATO, Takeshi**
  **Kamiina-gun, Nagano 399-4598 (JP)**
- **MORITANI, Tatsuru**
  **Tokyo 143-8555 (JP)**
- **MORINAGA, Tadahiko**
  **Tokyo 143-8555 (JP)**
- **SATO, Yuichi**
  **Tokyo 143-8555 (JP)**
- **KOTSUGAI, Akihiro**
  **Tokyo 143-8555 (JP)**

(74) Representative: **Fairbairn, Angus Chisholm**
**Marks & Clerk LLP**
**15 Fetter Lane**
**London EC4A 1BW (GB)**

(54) **PHARMACEUTICAL COMPOSITION**

(57) A pharmaceutical composition including particles each containing a water-soluble base material and a poorly water-soluble compound, the water-soluble base material containing a rapidly water-soluble compound, wherein the poorly water-soluble compound is a kinase inhibitor and exists in an amorphous state in the water-soluble base material.

FIG. 12

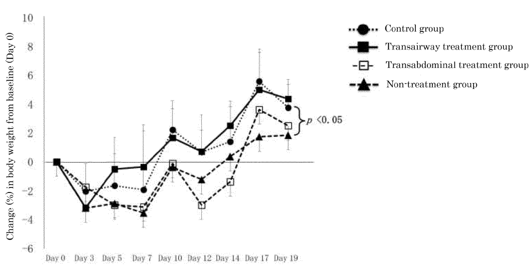


EP 3 991 750 A1

## Description

Technical Field

The present invention relates to a pharmaceutical composition.

Background Art

Currently, compounds that have been newly developed as medicines include many compounds having a considerably poor solubility in water, so-called poorly water-soluble compounds.

When such a poorly water-soluble compound is orally administered, the poorly water-soluble compound as a medicine is not sufficiently dissolved in the body, which may result in a decrease in bioavailability. In order to avoid the decrease in bioavailability, various approaches to dissolve the poorly water-soluble compound have been performed. For example, improvement in a dissolution rate achieved by forming a pharmaceutical agent that is the poorly water-soluble compound into nanoparticles to increase a surface area of the particles of the pharmaceutical agent and use of a solubilizer exhibiting solubilization in the pharmaceutical agent in combination have been investigated. Particularly, many particles improved in solubility, which are obtained by introducing a pharmaceutical agent into an inert base material, have been investigated.

For example, a solid pharmaceutical preparation that instantly releases a pharmaceutical compound with a low solubility by incorporating the pharmaceutical compound dissolved in a solubilizer into the solid pharmaceutical preparation has been proposed (see, for example, Patent document 1).

A solid dispersing element improved in solubility of a poorly water-soluble compound obtained by including, for example, a water-soluble polymer, a water-soluble saccharide, and a surfactant has been proposed (see, for example, Patent document 2).

As described above, various approaches to improve solubility of a poorly water-soluble pharmaceutical compound have been performed.

Citation List

Patent Document

Patent document 1: Japanese Patent No. 2960169
Patent document 2: Japanese Patent No. 5484910

Summary of Invention

Technical Problem

The present invention has an object to provide a pharmaceutical composition that can rapidly dissolve a poorly water-soluble compound.

Solution to Problem

A pharmaceutical composition of the present invention as a means for solving the problem includes particles each containing a water-soluble base material and a poorly water-soluble compound. The water-soluble base material contains a rapidly water-soluble compound. The poorly water-soluble compound is a kinase inhibitor and exists in an amorphous state in the water-soluble base material.

Effects of the Invention

According to the present invention, it is possible to provide a pharmaceutical composition that can rapidly dissolve a poorly water-soluble compound.

Brief Description of the Drawings

FIG. 1 is a cross-sectional view presenting one example of a liquid droplet forming unit.
FIG. 2 is a cross-sectional view presenting one example of a liquid column resonance droplet-discharging unit.
FIG. 3A is a schematic view presenting one example of a structure of a discharging hole.
FIG. 3B is a schematic view presenting another example of a structure of a discharging hole.
FIG. 3C is a schematic view presenting another example of a structure of a discharging hole.
FIG. 3D is a schematic view presenting another example of a structure of a discharging hole.
FIG. 4A is a schematic view presenting a standing wave of velocity fluctuation and a standing wave of pressure fluctuation when N = 1 and one end is fixed.
FIG. 4B is a schematic view presenting a standing wave of velocity fluctuation and a standing wave of pressure fluctuation when N = 2 and both ends are fixed.
FIG. 4C is a schematic view presenting a standing wave of velocity fluctuation and a standing wave of pressure fluctuation when N = 2 and both ends are free.
FIG. 4D is a schematic view presenting a standing wave of velocity fluctuation and a standing wave of pressure fluctuation when N = 3 and one end is fixed.
FIG. 5A is a schematic view presenting a standing wave of velocity fluctuation and a standing wave of pressure fluctuation when N = 4 and both ends are fixed.
FIG. 5B is a schematic view presenting a standing wave of velocity fluctuation and a standing wave of pressure fluctuation when N = 4 and both ends are free.
FIG. 5C is a schematic view presenting a standing wave of velocity fluctuation and a standing wave of pressure fluctuation when N = 5 and one end is fixed.
FIG. 6A is a schematic view presenting one exemplary pressure and velocity waveforms in a liquid-column-resonance liquid chamber when liquid droplets are discharged.
FIG. 6B is a schematic view presenting another exemplary pressure and velocity waveforms in a liquid-column-resonance liquid chamber when liquid droplets are discharged.
FIG. 6C is a schematic view presenting another exemplary pressure and velocity waveforms in a liquid-column-resonance liquid chamber when liquid droplets are discharged.
FIG. 6D is a schematic view presenting another exemplary pressure and velocity waveforms in a liquid-column-resonance liquid chamber when liquid droplets are discharged.
FIG. 6E is a schematic view presenting another exemplary pressure and velocity waveforms in a liquid-column-resonance liquid chamber when liquid droplets are discharged.
FIG. 7 is an image presenting exemplary actual liquid droplets discharged by a liquid droplet forming unit.
FIG. 8 is a graph presenting dependency of a liquid droplet-discharging velocity on a driving frequency.
FIG. 9 is a schematic view presenting one exemplary particle production apparatus.
FIG. 10 is a schematic view presenting one exemplary gas flow path.
FIG. 11 is a graph presenting results of a solubility test in Test Example 1.
FIG. 12 is a graph presenting results obtained by studying change in body weight of mice in Test Example 2.
FIG. 13A is Figure-1 presenting results obtained by studying a respiratory function test (maximal inspiratory capacity) in Test Example 2.
FIG. 13B is Figure-1 presenting results obtained by studying a respiratory function test (lung thorax compliance) in Test Example 2.
FIG. 13C is Figure-1 presenting results obtained by studying a respiratory function test (lung tissue elastance) in Test Example 2.
FIG. 13D is Figure-1 presenting results obtained by studying a respiratory function test (static lung compliance) in Test Example 2.
FIG. 14A is Figure-2 presenting results obtained by studying a respiratory function test (maximal inspiratory capacity) in Test Example 2.
FIG. 14B is Figure-2 presenting results obtained by studying a respiratory function test (lung thorax compliance) in Test Example 2.
FIG. 14C is Figure-2 presenting results obtained by studying a respiratory function test (lung tissue elastance) in Test Example 2.
FIG. 14D is Figure-2 presenting results obtained by studying a respiratory function test (static lung compliance) in Test Example 2.

Mode for Carrying out the Invention

(Functional particles)

Functional particles of the present invention each contain a water-soluble base material and a poorly water-soluble compound. The water-soluble base material contains a rapidly water-soluble compound. The poorly water-soluble compound exists in an amorphous state in the water-soluble base material. The functional particles are typically instantly soluble particles that rapidly dissolve in water or physiological saline. The functional particles of the present invention may further contain other ingredients, if necessary.

The functional particles of the present invention, typically instantly soluble particles, can be suitably produced by the below-described method of the present invention for producing functional particles.

In the present invention, the "functional particles" refer to a population of particulate compositions each containing a base material and a physiologically active substance and having a predetermined function. Examples thereof include, but are not limited to, DDS particles, sustained-release particles, and soluble particles. The functional particles may have two or more functions at the same time. For example, the functional particles may be DDS particles and sustained-release particles. In the present invention, the "instantly soluble particles" refer to, especially, particles where a physiologically active substance is allowed to instantly dissolve, among the soluble particles; i.e., functional particles that allow a physiologically active substance with no instant solubility to be instantly soluble. Typically, they refer to particles that when added to water or physiological saline, dissolve in the water or physiological saline, to be able to obtain a solution or dispersion liquid of a physiologically active substance contained in the particles. The physiologically active substance contained in the instantly soluble particles is typically a poorly water-soluble compound. The "being rapidly dissolved" or the "instantly soluble" may be different depending on a size of a particle, a temperature of a solvent, and solubility of a compound, but can be evaluated by using various methods known in the art (e.g., measurement of dissolution time). One example of the specific evaluation methods is, but is not limited to, the following method. Particles to be evaluated are added to, for example, water or a physiological saline solution so that the concentration of the poorly water-soluble compound reaches a certain concentration (e.g., 1% by mass). The resultant is shaken or stirred at a constant pace (e.g., two times per second). The time taken for the particles to completely dissolve is measured. For example, such particles that are completely dissolved to an extent that the particles cannot be visually confirmed within a certain time (e.g., within 30 minutes, within 20 minutes, within 10 minutes, within 5 minutes, within 3 minutes, within 2 minutes, within 1 minute, within 50 seconds, within 40 seconds, within 30 seconds, within 20 seconds, and within 10 seconds) is evaluated as being rapidly dissolved or being instantly soluble. When the particles are instantly soluble, they do not typically require special operation for dissolution (for example, continuous stirring over several hours, and atomization by using a homogenizer). In the present invention, the "being completely dissolved" or the "complete dissolution" refers typically to a state where no residue can be confirmed through, for example, visual observation. For example, the complete dissolution can be determined when no residue can be confirmed through, for example, visual observation and no substantial change in the concentration of a solution even if the solution continues to be stirred for a certain period of time (e.g., for 15 minutes).

In the present invention, the "rapidly water-soluble compound" refers to a compound that has a property of being rapidly dissolved in water with only short-time stirring or shaking within a certain time (e.g., within 1 minute, within 50 seconds, within 40 seconds, within 30 seconds, within 20 seconds, and within 10 seconds) without performing special operation for dissolution when the rapidly water-soluble compound is added to water. Examples of the rapidly water-soluble compound include, but are not limited to, low-molecular saccharides (e.g., monosaccharides and disaccharides), oligosaccharides, reducing sugars, and sugar alcohols. The rapidly water-soluble compound that can be used in the present invention is preferably a solid at normal temperature.

As a result of diligent studies to rapidly dissolve a compound exhibiting a poor water-solubility, the present inventors obtained the following finding. Specifically, when microparticles containing a poorly water-soluble compound are produced by using, as a base material, a substance (e.g., monosaccharide and disaccharide) that is rapidly dissolved in water, the microparticles are rapidly dissolved in water to form an aqueous solution of the poorly water-soluble compound.

The functional particles of the present invention are in an amorphous state. Without being bound by any particular theory, it is understood that the instantly soluble particles of the present invention has a solid dispersion structure in which an amorphous poorly water-soluble compound is dispersed in an amorphous water-soluble base material. Therefore, when the functional particles of the present application, typically instantly soluble particles, are added to water or physiological saline, the surrounding water-soluble base material is rapidly dissolved to thereby rapidly disperse, in water or physiological saline, the poorly water-soluble compound in a dispersed state. Moreover, it is deemed that because both the water-soluble base material and the poorly water-soluble compound are amorphous, the water-soluble base material and the poorly water-soluble compound in an amorphous state are more energetically unstable than those in a crystalline state, and thus rapid dissolution in water or physiological saline can be achieved.

-Water-soluble base material-

The water-soluble base material is not particularly limited as long as the water-soluble base material itself is rapidly dissolved in water and can be dispersed in a base material without chemically reacting with the poorly water-soluble compound. Examples of the water-soluble base material include rapidly water-soluble compounds.

Examples of the rapidly water-soluble compound include monosaccharides, disaccharides, oligosaccharides, reducing sugars, and sugar alcohols.

Examples of the monosaccharide include glucose, mannose, idose, galactose, fucose, ribose, and xylose.

Examples of the disaccharide include lactose, sucrose, maltose, and trehalose.

Examples of the oligosaccharide include raffinose (trisaccharide), maltotriose (trisaccharide), and acarbose (tetrasaccharide).

Examples of the reducing sugar include turanose.

Examples of the sugar alcohol include glycerin, erythritol, xylitol, lactitol, sorbitol, maltitol, and mannitol.

Among them, monosaccharides, disaccharides, or both are preferable, and lactose is most preferable.

The water-soluble base material usable may be a hydrate.

Since the functional particles of the present invention contain the water-soluble base material, it is possible to increase solubility of the bellow-described poorly water-soluble compounds in water, and wettability of instantly soluble particles in a solvent during preparation of a solution as well as wettability of the poorly water-soluble compounds contained in the functional particles.

An amount of the water-soluble base material in the functional particles is not particularly limited and may be appropriately selected depending on the intended purpose as long as the amount is such an amount that the water-soluble base material can exhibit a function of rapidly dissolving the poorly water-soluble compounds in water. The amount thereof is preferably 10% by mass or greater but 99.9% by mass or less, more preferably 30% by mass or greater but 80% by mass or less, further preferably 50% by mass or greater but 80% by mass or less.

-Poorly water-soluble compound-

The poorly water-soluble compound refers to a compound having a water/octanol partition coefficient (logP value) of 3 or more.

The water/octanol partition coefficient refers to a ratio between a concentration of a compound dissolved in an aqueous phase and a concentration of the compound dissolved in an octanol phase in a two-phase system of water and octanol, and is generally represented by $Log_{10}$ (concentration of compound in octanol phase / concentration of compound in aqueous phase).

A method for measuring the water/octanol partition coefficient (logP value) can be any known method in the art. Examples of the method include the method described in JIS Z 7260-107.

The poorly water-soluble compound is not particularly limited and may be appropriately selected depending on the intended purpose, as long as it has a water/octanol partition coefficient (logP value) of 3 or more. For example, the poorly water-soluble compound is preferably a physiologically active substance. In the present invention, the "physiologically active substance" refers to an active ingredient that is used for allowing living bodies to exhibit a physiological effect. The "physiological effect" refers to an effect occurring when the physiologically active substance exhibits a physiological activity at the intended site. The "physiological activity" means that the physiologically active substance acts on the intended site (e.g., the target tissue) to give changes and impacts thereto.

The physiologically active substance is not particularly limited and may be appropriately selected depending on the intended purpose. Examples of the physiologically active substance include pharmaceutical compounds, cosmetic compounds, and functional food compounds. Pharmaceutical compounds are preferable. The pharmaceutical compound may be any compound known as an active ingredient of a drug. Not only low-molecular-weight pharmaceutical compounds but also polypeptides, nucleic acids, etc. can be used. In the present invention, the "polypeptide" refers to a substance having two or more peptide bonds (amide bonds) in a molecule thereof. The "polypeptide" includes not only those including two or more amino acids linked together via peptide bonds but also glycopeptide-based antibiotics, cyclic polypeptide-based antibiotics, etc. In particular, the polypeptides having specific conformations to have predetermined properties are referred to as a "protein". Preferable low-molecular-weight pharmaceutical compounds are, among others, kinase inhibitors including, for example, a tyrosine kinase inhibitor and serine/threonine kinase inhibitor. The polypeptide is preferably an antibody, an enzyme, etc., among others. The nucleic acid is preferably an antisense nucleic acid, etc., among others. In the present invention, describing a pharmaceutical compound as a compound name is intended to include not only the described compound but also any pharmaceutically acceptable forms of the compound, such as salts, solvates, or stereoisomers thereof.

Examples of the kinase inhibitor include nintedanib, afatinib, gefitinib, erlotinib, osimertinib, bosutinib, vandetanib, alectinib, lorlatinib, abemaciclib, tyrphostin AG494, sorafenib, dasatinib, lapatinib, imatinib, motesanib, lestaurtinib, tandutinib, dorsomorphin, axitinib, and 4-benzyl-2-methyl-1,2,4-thiadiazolidine-3,5-dione.

Examples of the polypeptide include ciclosporin, vancomycin, teicoplanin, and daptomycin.

The drug is preferably a kinase inhibitor or an antibiotics.

Examples of other poorly water-soluble compounds include quercetin, testosterone, indomethacin, tranilast, and tacrolimus.

An amount of the poorly water-soluble compound in the functional particles is not particularly limited and may be appropriately selected depending on the intended purpose, as long as an effect of the poorly water-soluble compound can be sufficiently achieved and the amount falls within a range where the poorly water-soluble compound can be dissolved in water or physiological saline. As the amount of the poorly water-soluble compound increases, the solubility decreases accordingly. In one embodiment, the amount of the poorly water-soluble compound can be, for example, 75% by mass or less, preferably 0.01% by mass or more but 75% by mass or less, more preferably 1% by mass or more but 75% by mass or less, further preferably 10% by mass or more but 70% by mass or less, particularly preferably 20% by mass or more but 50% by mass or less. When the amount of the poorly water-soluble compound is 0.01% by mass or more, an amount of a solution of the instantly soluble particles required to administer a required amount of a pharmaceutical agent can be decreased. Meanwhile, when the amount of the poorly water-soluble compound is 75% by mass or less, it is possible to ensure a high instant solubility of the poorly water-soluble compound.

When the amount of the poorly water-soluble compound is less than 0.01% by mass, a concentration of the pharmaceutical agent is decreased at the time of taking the pharmaceutical agent. As a result, a larger amount of the solution should be administered, which is not efficient. Meanwhile, the amount of the poorly water-soluble compound is more than 75% by mass, an effect of instant solubility of the pharmaceutical agent is decreased.

As described above, the functional particles of the present invention in one embodiment include a pharmaceutical compound, and are particularly in the form where a poorly water-soluble pharmaceutical compound can be rapidly dissolved in water or physiological saline. Therefore, the particles of the present invention can be particularly suitably used in an administration form of a pharmaceutical composition that is used by being dissolved in water or physiological saline. In addition, it can be suitably used as a pharmaceutical composition that can be prepared at the time of use.

A volume average particle diameter (Dv) of the functional particle is preferably 0.5 μm or more but 50 μm or less, more preferably 0.5 μm or more but 20 μm or less. When the volume average particle diameter (Dv) of the functional particles is 0.5 μm or more but 50 μm or less, the poorly water-soluble compound contained in the functional particles is easily included in the particles in an amorphous state, which increases solubility of the poorly water-soluble compound.

In one embodiment, the functional particles have a relative span factor (R.S.F) that satisfies the following expression (1).

$$0<(\mathrm{R.S.F}) \leqq 1.5 \cdots \text{Expression (1)}$$

The (R.S.F) is defined as (D90-D10)/D50.

The D90 denotes a cumulative 90% by volume from a small particle side of a cumulative particle size distribution, the D50 denotes a cumulative 50% by volume from the small particle side of the cumulative particle size distribution, and the D10 denotes a cumulative 10% by volume from the small particle side of the cumulative particle size distribution. The upper limit of the R.S.F. is not particularly limited. Examples of the upper limit include 1.5, 1.4, 1.3, 1.2, 1.1, 1.0, 0.9, 0.8, 0.7, 0.6, and 0.5.

The (R.S.F) can be measured by, for example, a laser diffraction / scattering particle size distribution analyzer (device name: MICROTRAC MT3000II, available from MicrotracBEL Corp.) or a fiber-optics particle analyzer ("FPAR-1000", available from Otsuka Electronics Co., Ltd.) using the dynamic light scattering method.

-Other ingredients-

The other ingredients are not particularly limited and may be appropriately selected depending on the intended purpose. Examples of the other ingredients include those described in the section --Other ingredients-- of the below-described (Method for producing functional particles and apparatus for producing functional particles).

(Method for producing functional particles and apparatus for producing functional particles)

A method of the present invention for producing functional particles includes: a liquid droplet forming step of discharging a liquid containing a rapidly water-soluble compound and a poorly water-soluble compound from a discharging hole to form liquid droplets; and a particle forming step of solidifying the liquid droplets to form particles. The method of the present invention further includes other steps, if necessary.

An apparatus of the present invention for producing functional particles includes: a liquid droplet forming unit configured to discharge a liquid containing a rapidly water-soluble compound and a poorly water-soluble compound from a discharging hole to form liquid droplets; and a particle forming unit configured to solidify the liquid droplets to form the particles. The apparatus of the present invention further includes a particle collecting unit and other units, if necessary.

<Liquid droplet forming step and liquid droplet forming unit>

The liquid droplet forming step is a step of discharging a liquid containing the rapidly water-soluble compound and the poorly water-soluble compound (hereinafter this liquid may be referred to as a "particle composition liquid") from a discharging hole to form liquid droplets, and is performed by the liquid droplet forming unit.

-Particle composition liquid-

The particle composition liquid contains, in a solvent, the water-soluble base material and the poorly water-soluble compound. The particle composition liquid further contains other ingredients, if necessary.

--Solvent--

The solvent is not particularly limited and may be appropriately selected depending on the intended purpose. Preferable examples of the solvent include those that can dissolve or disperse the water-soluble base material and the poorly water-soluble compound, or a pharmaceutical acceptable salt thereof. In order to simultaneously dissolve the water-soluble base material and the poorly water-soluble compound, two or more kinds of solvents are preferably mixed for use.

Examples of the solvent include water, aliphatic halogenated hydrocarbons (e.g., dichloromethane, dichloroethane, and chloroform), alcohols (e.g., methanol, ethanol, and propanol), ketones (e.g., acetone and methyl ethyl ketone), ethers (e.g., diethyl ether, dibutyl ether, and 1,4-dioxane), aliphatic hydrocarbons (e.g., n-hexane, cyclohexane, and n-heptane), aromatic hydrocarbons (e.g., benzene, toluene, and xylene), organic acids (e.g., acetic acid and propionic acid), esters (e.g., ethyl acetate), amides (e.g., dimethylformamide and dimethylacetamide), and mixture solvents thereof.

An amount of the solvent is preferably 70% by mass or more but 99.5% by mass or less, more preferably 90% by mass or more but 99% by mass or less, relative to a total amount of the liquid. When the amount of the solvent is 70% by mass or more but 99.5% by mass or less relative to the total amount of the liquid, production stability can be improved because solubility of the poorly water-soluble compound and viscosity of the liquid can be appropriate.

--Rapidly water-soluble compound--

The rapidly water-soluble compound is the same that can be used in the functional particles of the present invention.

An amount of the rapidly water-soluble compound is preferably 0.1% by mass or more but 20.0% by mass or less, more preferably 0.1% by mass or more but 15.0% by mass or less, relative to the total amount of the particle composition liquid.

--Poorly water-soluble compound--

The poorly water-soluble compound is the same that can be used in the functional particles of the present invention.

An amount of the poorly water-soluble compound is preferably 0.005% by mass or more but 5.0% by mass or less, more preferably 0.05% by mass or more but 5.0% by mass or less, further preferably 0.1% by mass or more but 3.0% by mass or less, relative to the total amount of the particle composition liquid.

--Other ingredients--

The other ingredients are not particularly limited and may be appropriately selected depending on the intended purpose. They are preferably those that can conventionally be used in drugs.

Examples of the other ingredients include water, an excipient, a flavoring agent, a disintegrating agent, a fluidizer, an adsorbent, a lubricant, an odor-masking agent, a surfactant, a perfume, a colorant, an anti-oxidant, a masking agent, an anti-static agent, and a humectant. These may be used alone or in combination.

The excipient is not particularly limited and may be appropriately selected depending on the intended purpose. Examples of the excipient include lactose, sucrose, mannitol, glucose, fructose, maltose, erythritol, maltitol, xylitol, palatinose, trehalose, sorbitol, crystalline cellulose, talc, silicic anhydride, anhydrous calcium phosphate, precipitated calcium carbonate, and calcium silicate. These may be used alone or in combination.

The flavoring agent is not particularly limited and may be appropriately selected depending on the intended purpose. Examples of the flavoring agent include L-menthol, sucrose, D-sorbitol, xylitol, citric acid, ascorbic acid, tartaric acid, malic acid, aspartame, acesulfame potassium, thaumatin, saccharin sodium, dipotassium glycyrrhizate, sodium glutamate, sodium 5'-inosinate, and sodium 5'-guanylate. These may be used alone or in combination.

The disintegrating agent is not particularly limited and may be appropriately selected depending on the intended purpose. Examples of the disintegrating agent include low-substituted hydroxypropylcellulose, carmellose, carmellose calcium, carboxymethyl starch sodium, croscarmellose sodium, crospovidone, hydroxypropyl starch, and corn starch. These may be used alone or in combination.

The fluidizer is not particularly limited and may be appropriately selected depending on the intended purpose. Examples of the fluidizer include light anhydrous silicic acid, hydrated silicon dioxide, and talc. These may be used alone or in combination.

As the light anhydrous silicic acid, a commercially available product can be used. The commercially available product of light anhydrous silicic acid is not particularly limited and may be appropriately selected depending on the intended purpose. Examples of the commercially available product of light anhydrous silicic acid include ADSOLIDER 101 (available from Freund Corporation: average pore diameter: 21 nm).

As the adsorbent, a commercially available product can be used. The commercially product of the adsorbent is not particularly limited and may be appropriately selected depending on the intended purpose. Examples of the commercially product of the adsorbent include product name: CARPLEX (ingredient name: synthetic silica, registered trademark of Evonik Japan), product name: AEROSIL (registered trademark of NIPPON AEROSIL CO., LTD.) 200 (ingredient name: hydrophilic fumed silica), product name: SYLYSIA (ingredient name: amorphous silicon dioxide, registered trademark of Fuji Silysia chemical Ltd.), and product name: ALCAMAC (ingredient name: synthetic hydrotalcite, registered trademark of Kyowa Chemical Industry Co., Ltd.). These may be used alone or in combination.

The lubricant is not particularly limited and may be appropriately selected depending on the intended purpose. Examples of the lubricant include magnesium stearate, calcium stearate, sucrose fatty acid ester, sodium stearyl fumarate, stearic acid, polyethylene glycol, and talc. These may be used alone or in combination.

The odor-masking agent is not particularly limited and may be appropriately selected depending on the intended purpose. Examples of the odor-masking agent include trehalose, malic acid, maltose, potassium gluconate, anise essential oil, vanilla essential oil, and cardamom essential oil. These may be used alone or in combination.

The surfactant is not particularly limited and may be appropriately selected depending on the intended purpose. Examples of the surfactant include Polysorbates (e.g., Polysorbate 80); polyoxyethylene-polyoxypropylene copolymer; and sodium lauryl sulfate. These may be used alone or in combination.

The perfume is not particularly limited and may be appropriately selected depending on the intended purpose. Examples of the perfume include lemon oil, orange oil, and peppermint oil. These may be used alone or in combination.

The colorant is not particularly limited and may be appropriately selected depending on the intended purpose. Examples of the colorant include titanium oxide, Food Yellow No. 5, Food Blue No. 2, Ferric oxide, and Yellow Ferric Oxide. These may be used alone or in combination.

The anti-oxidant is not particularly limited and may be appropriately selected depending on the intended purpose. Examples of the anti-oxidant include sodium ascorbate, L-cysteine, sodium sulfite, and vitamin E. These may be used alone or in combination.

The masking agent is not particularly limited and may be appropriately selected depending on the intended purpose. Examples of the masking agent include titanium oxide. These may be used alone or in combination.

The anti-static agent is not particularly limited and may be appropriately selected depending on the intended purpose. Examples of the anti-static agent include talc and titanium oxide. These may be used alone or in combination.

The humectant is not particularly limited and may be appropriately selected depending on the intended purpose. Examples of the humectant include Polysorbate 80, sodium lauryl sulfate, sucrose fatty acid ester, macrogol, and hydroxypropylcellulose (HPC). These may be used alone or in combination.

The particle composition liquid may not include a solvent as long as the liquid is in a state that the water-soluble base material and the poorly water-soluble compound are dissolved, the liquid is in a state that the poorly water-soluble compound is dispersed, or the liquid is a liquid when discharged. The liquid may be in a state that particle ingredients are melted.

-Discharging hole-

The discharging hole is not particularly limited and may be appropriately selected depending on the intended purpose. Examples of the discharging hole include an opening provided in, for example, a nozzle plate.

The number, a cross-sectional shape, and a size of the discharging holes may be appropriately selected.

The number of discharging holes is not particularly limited and may be appropriately selected depending on the intended purpose. For example, the number thereof is preferably 2 or more but 3,000 or less. When the number of discharging holes is 2 or more but 3,000 or less, productivity can be improved.

A cross-sectional shape of the discharging hole is not particularly limited and may be appropriately selected depending on the intended purpose. Examples of the cross-sectional shape include: (1) such a tapered shape that an opening diameter is decreased from a liquid contact surface (inlet) of a discharging hole toward a discharging hole (outlet); (2) such a shape that an opening diameter is narrowed from a liquid contact surface (inlet) of a discharging hole toward a discharging hole (outlet) while its round shape is maintained; (3) such a shape that an opening diameter is narrowed from a liquid contact surface (inlet) of a discharging hole toward a discharging hole (outlet) while a certain nozzle angle is maintained; and (4) combinations of the shape of (1) and the shape of (2). Among them, (3) such a shape that an opening diameter is narrowed from a liquid contact surface (inlet) of a discharging hole toward a discharging hole (outlet) while a certain nozzle angle is maintained is preferable because pressure to be applied to a liquid at the discharging hole reaches the maximum.

The nozzle angle in the shape of (3) is not particularly limited and may be appropriately selected depending on the intended purpose. The nozzle angle thereof is preferably 60° or more but 90° or less. When the nozzle angle is 60° or more, pressure is easily applied to a liquid, and processing is easily performed. When the nozzle angle is 90° or less, pressure can be applied at the discharging hole to stabilize discharging of liquid droplets. Therefore, the maximum value of the nozzle angle is preferably 90°.

A size of the discharging hole may be appropriately selected considering the sustained-releasability of particles to be produced. For example, a diameter of the discharging hole is preferably 12 μm or more but 100 μm or less, more preferably 15 μm or more but 30 μm or less. When the size of the discharging hole is 12 μm or more but 100 μm or less, it is possible to obtain particles having such a sufficient particle diameter that achieves sustained-releasability.

<<Liquid droplet forming unit»

The liquid droplet forming unit is not particularly limited and a known liquid droplet forming unit may be appropriately used depending on the intended purpose. Examples of the liquid droplet forming unit include spray nozzles, one-fluid nozzles, two-fluid nozzles, film vibration-type discharging units, Rayleigh-breakup-type discharging units, liquid vibration-type discharging units, and liquid column resonance-type discharging units.

Examples of the film vibration-type discharging unit include discharging units described in Japanese Unexamined Patent Application Publication No. 2008-292976. Examples of the Rayleigh-breakup-type discharging unit include discharging units described in Japanese Patent No. 4647506. Examples of the liquid vibration-type discharging unit include discharging units described in Japanese Unexamined Patent Application Publication No. 2010-102195.

In order to narrow the particle size distribution of the liquid droplet and ensure productivity of the instantly soluble particles, it is possible to employ liquid column resonance for forming liquid droplets with the liquid column resonance-type discharging unit. In the liquid column resonance for forming liquid droplets, vibration may be imparted to a liquid in a liquid-column-resonance liquid chamber to form standing waves through liquid column resonance, to discharge the liquid from a plurality of the discharging holes formed to regions that correspond to anti-nodes of the standing waves.

Examples of the liquid discharged by the liquid droplet forming unit in the present invention include an embodiment of a "particle ingredient-containing liquid" in which particle ingredients to be obtained are dissolved or dispersed. The liquid may not include a solvent as long as it is a liquid when discharged, and may be an embodiment of a "particle ingredient-melted liquid" in which the particle ingredients are melted.

<Particle forming step>

The particle forming step is a step of removing the solvent from the liquid droplets formed in the liquid droplet forming step, to form particles.

Specifically, the particle forming step is a step of solidifying liquid droplets of the particle composition liquid containing the rapidly water-soluble compound and the poorly water-soluble compound discharged into a gas from the liquid droplet forming unit.

The particle forming unit is a unit configured to solidify the liquid droplets to form the particles.

«Particle forming unit»

Formation of particles of the liquid droplets may be performed with any unit and may be appropriately selected depending on characteristics of the particle composition liquid as long as the solvent can be removed from the liquid droplets. For example, when the particle composition liquid obtained by dissolving or dispersing a solid raw material in a volatile solvent is used, liquid droplets are discharged, and the liquid droplets are discharged into a conveyance gas flow, followed by drying. That is, solidification of the liquid droplets can be achieved by discharging the liquid droplets into the conveyance gas flow and volatilizing the solvent in the liquid droplets. In order to dry the solvent, a drying condition can be adjusted by appropriately selecting a temperature and a vapor pressure of a gas to be discharged and kinds of gases. Even when the solvent is not completely dried, additional drying may be performed in another step after collecting, as long as collected particles are kept solid. In addition, a solidification condition may be achieved through a change of temperatures or chemical reaction.

The conveyance gas flow prevents a decrease in the liquid droplet-discharging velocity immediately after the liquid droplet is discharged, and suppresses cohesion (unification) of the liquid droplets. The conveyance gas flow is provided for the following reasons.

When discharged liquid droplets contact with each other before the liquid droplets are dried, the liquid droplets are unified to form one liquid droplet (hereinafter, this phenomenon is referred to as coalescence). In order to obtain particles having a uniform (narrow) particle size distribution, it is necessary to maintain a certain distance between the discharged droplets. However, the discharged liquid droplet travels at a certain initial velocity, but the velocity of the liquid droplet is decreased soon due to air resistance. The liquid droplet decreased in the velocity is caught up with by a liquid droplet subsequently discharged, which leads to coalescence. This phenomenon occurs regularly, and thus particle size distribution of the resultant particles are not uniform (narrow). In order to prevent coalescence of the liquid droplets, it is necessary to prevent a decrease in the liquid droplet-discharging velocity, and to solidify/convey the liquid droplet while coalescence of the liquid droplets is prevented by means of conveyance gas flow so that the liquid droplets do not contact with each other.

A method for solidifying the liquid droplet using the conveyance gas flow is not particularly limited and may be appropriately selected depending on the intended purpose. Preferable examples of the method include a method where a conveyance direction of the conveyance gas flow is a substantially vertical direction to a direction in which the liquid droplet is to be discharged. The drying method using the conveyance gas flow will be described in detail in the description of drawings that will be described hereinafter.

In order to dry the solvent, it is preferable to adjust, for example, the temperature and the vapor pressure of the conveyance gas flow, and kinds of gasses.

As long as collected particles are kept solid, even when the collected particle are not completely dried, a drying step may be additionally provided in another step after the collecting.

In addition, a method for drying the liquid droplet by application of a temperature change or a chemical change may be used.

<Other steps>

The other steps are not particularly limited and may be appropriately selected depending on the intended purpose. Examples of the other steps include a particle collecting step.

The particle collecting step is a step of collecting dried particles and can be suitably performed by a particle collecting unit.

The particle collecting unit is not particularly limited and may be appropriately selected depending on the intended purpose. Examples of the article collecting unit include cyclone collection and bag filters.

A method of the present invention for producing functional particles can be suitably performed by an apparatus for producing a functional particle.

Here, the apparatus for producing functional particles will be described.

FIG. 1 is a schematic cross-sectional view of the liquid droplet forming unit 11. The liquid droplet forming unit 11 includes a common liquid supplying path 17 and a liquid-column-resonance liquid chamber 18. The liquid-column-resonance liquid chamber 18 is connected to the common liquid supplying path 17 disposed on one of wall surfaces at both side wall surfaces in a longitudinal direction. Moreover, the liquid-column-resonance liquid chamber 18 includes a discharging hole 19 and a vibration generating unit 20. The discharging hole 19 is configured to discharge liquid droplets 21, and is disposed on one wall surface of the wall surfaces connected to the side wall surfaces. The vibration generating unit 20 is configured to generate high frequency vibration to form liquid column resonance standing waves, and is disposed on the wall surface facing the discharging hole 19. Note that, a high frequency power source, which is not presented, is coupled to the vibration generating unit 20. In FIG. 1, the reference numeral 9 denotes an elastic plate, the reference numeral 12 denotes a gas flow path, and the reference numeral 14 denotes liquid.

FIG. 2 is a schematic cross-sectional view of another example of a liquid droplet forming unit. FIG. 2 presents a liquid column resonance droplet-discharging unit 10 including the liquid droplet forming unit presented in FIG. 1. The liquid 14 is allowed to pass through the liquid supplying pipe by a liquid circulating pump that is not presented to flow into the common liquid supplying path 17 of the liquid column resonance droplet-discharging unit 10 presented in FIG. 2. Then, the liquid 14 passes through the liquid supplying path 17a of the liquid droplet forming unit 11 presented in FIG. 1 from the common liquid supplying path 17 and is supplied to the liquid-column-resonance liquid chamber 18. Within the liquid-column-resonance liquid chamber 18 charged with the liquid 14, a pressure distribution is formed by liquid column resonance standing waves generated by the vibration generating unit 20. Then, liquid droplets 21 are discharged from the discharging hole 19 disposed in the regions that correspond to anti-nodes of the standing waves and are the sections where the amplitude of the liquid column resonance standing waves is large and pressure displacement is large. The regions corresponding to anti-nodes of the standing waves owing to the liquid column resonance are regions other than nodes of the standing waves. The regions are preferably regions each having sufficiently large amplitude enough to discharge the liquid through the pressure displacement of the standing waves, more preferably regions having a width corresponding to ±1/4 of a wavelength from a position of a local maximum amplitude of a pressure standing wave (i.e., a node of a velocity standing wave) toward positions of a local minimum amplitude.

Even when there are a plurality of openings of the discharging hole, substantially uniform liquid droplets can be formed from the openings as long as the openings of the discharging hole are disposed in the regions corresponding to the anti-nodes of the standing waves. Moreover, discharging of the liquid droplets can be performed efficiently, and clogging of the discharging hole is unlikely to occur. Note that, the liquid 14 passing through the common liquid supplying path 17 travels through a liquid returning pipe (not presented) to be returned to the raw material housing container. Once the amount of the liquid 14 inside the liquid-column-resonance liquid chamber 18 is reduced by discharging of the liquid droplets 21, a flow rate of the liquid 14, which is supplied from the liquid supplying path 17a by suction power generated by the action of the liquid column resonance standing waves inside the liquid-column-resonance liquid chamber 18, is increased. As a result, the liquid-column-resonance liquid chamber 18 is refilled with the liquid 14. When the liquid-column-resonance liquid chamber 18 is refilled with the liquid 14, the flow rate of the liquid 14 passing through the liquid supplying path 17a returns to the previous flow rate.

The liquid-column-resonance liquid chamber 18 of the liquid droplet forming unit 11 is formed by joining frames with each other. The frames are formed of materials having high stiffness to the extent that a resonance frequency of the liquid is not influenced at a driving frequency (e.g., metals, ceramics, and silicones). As presented in FIG. 1, a length L between the side wall surfaces of the liquid-column-resonance liquid chamber 18 in a longitudinal direction is determined based on the principle of the liquid column resonance described below. Moreover, a width W of the liquid-column-resonance liquid chamber 18 presented in FIG. 2 is preferably smaller than half of the length L of the liquid-column-resonance liquid chamber 18 so that excess frequency is not given to liquid column resonance. Moreover, a plurality of the liquid-column-resonance liquid chambers 18 are preferably disposed per one liquid droplet forming unit 10 in order to drastically improve productivity. The number of the liquid-column-resonance liquid chambers 18 is not particularly limited and may be appropriately selected depending on the intended purpose. The number of the liquid-column-resonance liquid chambers 18 is preferably 100 or greater but 2,000 or less in order to achieve both productivity and operability. In each liquid-column-resonance liquid chamber 18, the common liquid supplying path 17 is coupled to and connected to the liquid supplying path 17a configured to supply the liquid. The liquid supplying path 17a is coupled to a plurality of the liquid-column-resonance liquid chambers 18.

Moreover, the vibration generating unit 20 of the liquid droplet forming unit 11 is not particularly limited as long as the vibration generating unit 20 is driven at a predetermined frequency. The vibration generating unit is preferably formed by attaching a piezoelectric material onto an elastic plate 9. The frequency is preferably 150 kHz or greater, more preferably 300 kHz or greater but 500 kHz or less from the viewpoint of productivity. The elastic plate constitutes a portion of the wall of the liquid-column-resonance liquid chamber in a manner that the piezoelectric material does not come into contact with the liquid. The piezoelectric material may be, for example, a piezoelectric ceramic such as lead zirconate titanate (PZT), and is typically often laminated due to a small displacement amount. Other examples of the piezoelectric material include piezoelectric polymers (e.g., polyvinylidene fluoride (PVDF)) and monocrystals (e.g., crystal, $LiNbO_3$, $LiTaO_3$, and $KNbO_3$). The vibration generating unit 20 is preferably disposed per one liquid-column-resonance liquid chamber in a manner that the vibration generating unit 20 can individually control each liquid-column-resonance liquid chamber. It is preferable that the liquid-column-resonance liquid chambers be individually controlled via the elastic plates by partially cutting a block-shaped vibration generating unit, which is formed of one of the above-described materials, according to geometry of the liquid-column-resonance liquid chambers.

As presented in FIG. 2, a plurality of openings are formed in the discharging hole 19. In terms of high productivity, a structure, in which the discharging hole 19 is disposed in the width direction of the liquid-column-resonance liquid chamber 18, is preferably used. Moreover, the frequency of the liquid column resonance is desirably appropriately determined by checking discharging of liquid droplets, because the frequency of the liquid column resonance varies depending on the arrangement of opening of the discharging hole 19.

FIGs. 3A to 3D are schematic views presenting exemplary structures of discharging holes. As presented in FIGs. 3A to 3D, cross-sectional shapes of the discharging holes are presented as tapered shapes in which opening diameters gradually decrease from liquid contact surfaces (inlet) towards discharging holes (outlet) of the discharging holes. However, the cross-sectional shapes may be appropriately selected.

As presented in FIG. 3A, the discharging holes 19 have a shape in which an opening diameter gradually decreases from a liquid contact surface towards the discharging hole 19 of the discharging hole while its round shape is maintained. Such a shape can be the most preferable shape from the viewpoint of stable discharging because pressure applied to the liquid at the discharging hole is the largest.

As presented in FIG. 3B, the discharging holes 19 have a shape in which an opening diameter gradually decreases from a liquid contact surface towards a discharging hole 19 of the discharging hole while a certain angle is maintained. Such a shape makes it possible to appropriately change the nozzle angle 24. The shape described in FIG.

3B can increase pressure applied to the liquid adjacent to the discharging holes depending on the nozzle angle, similarly to the shape presented in FIG. 3A.

The nozzle angle 24 is not particularly limited and may be appropriately selected depending on the intended purpose, but is preferably 60 degrees or more but 90 degrees or less. When the nozzle angle is 60 degrees or more, pressure is easily applied to the liquid, resulting in easy processing. When the nozzle angle 24 is 90 degrees or less, pressure is applied adjacent to the outlets of the discharging holes, resulting in stable formation of the liquid droplets. Therefore, the maximum value of the nozzle angle 24 is preferably 90 degrees (corresponding to FIG. 3C).

In FIG. 3D, the discharging holes have a shape obtained by combining the shape presented in FIG. 3A with the shape presented in FIG. 3B. The shape of the discharging holes may be changed stepwise in this way.

A mechanism by which liquid droplets are formed by the liquid droplet forming unit based on the liquid column resonance will now be described.

Firstly, the principle of a liquid column resonance phenomenon that occurs in the liquid-column-resonance liquid chamber 18 of the liquid droplet forming unit 11 presented in FIG. 1 will be described.

A wavelength ($\lambda$) at which liquid resonance occurs is represented by Expression 1 below:

$$\lambda = c / f \cdots \text{(Expression 1)}$$

where c denotes sound velocity of the liquid in the liquid-column-resonance liquid chamber; and f denotes a driving frequency applied by the vibration generating unit 20 to the liquid serving as a medium.

In the liquid-column-resonance liquid chamber 18 in FIG. 1, a length from a frame end at a fixed end side to an end at the common liquid supplying path 17 side is represented as L. A height h1 (about 80 $\mu$m) of the frame end at the common liquid supplying path 17 side is about 2 times as high as a height h2 (about 40 $\mu$m) of a communication hole. The end at the common liquid supplying path side is assumed to be equivalent to a closed fixed end. In such cases where both ends are fixed, resonance is most efficiently formed when the length L corresponds to an even multiple of 1/4 of the wavelength ($\lambda$). This can be represented by Expression 2 below:

$$L = (N / 4)\,\lambda \cdots \text{(Expression 2)}$$

In the Expression 2, L denotes a length of the liquid-column-resonance liquid chamber in a longitudinal direction; N denotes an even number; and $\lambda$ denotes a wavelength at which liquid resonance occurs.

The Expression 2 is also satisfied when the both ends are free, that is, the both ends are completely opened.

Likewise, when one end is equivalent to a free end from which pressure is released and the other end is closed (fixed end), that is, when one of the ends is fixed or one of the ends is free, resonance is most efficiently formed when the length L corresponds to an odd multiple of 1/4 of the wavelength $\lambda$. That is, N in the Expression 2 denotes an odd number.

The most efficient driving frequency f is represented by Expression 3 which is derived from the Expression 1 and the Expression 2:

$$f = N \times c / (4L) \cdots \text{(Expression 3)}$$

In the Expression 3, L denotes a length of the liquid-column-resonance liquid chamber in a longitudinal direction; c denotes velocity of an acoustic wave of a liquid; and N denotes a natural number.

However, actually, vibration is not amplified unlimitedly because liquid has viscosity which attenuates resonance. Therefore, the resonance has a Q factor, and also occurs at a frequency adjacent to the most efficient driving frequency f calculated according to the Expression 3, as represented by Expression 4 and Expression 5 below.

FIG. 4A is a schematic view presenting a standing wave of velocity fluctuation and a standing wave of pressure fluctuation when N = 1 and one end is fixed.

FIG. 4B is a schematic view presenting a standing wave of velocity fluctuation and a standing wave of pressure fluctuation when N = 2 and both ends are fixed.

FIG. 4C is a schematic view presenting a standing wave of velocity fluctuation and a standing wave of pressure fluctuation when N = 2 and both ends are free.

FIG. 4D is a schematic view presenting a standing wave of velocity fluctuation and a standing wave of pressure fluctuation when N = 3 and one end is fixed.

FIG. 5A is a schematic view presenting a standing wave of velocity fluctuation and a standing wave of pressure fluctuation when N = 4 and both ends are fixed.

FIG. 5B is a schematic view presenting a standing wave of velocity fluctuation and a standing wave of pressure fluctuation when N = 4 and both ends are free.

FIG. 5C is a schematic view presenting a standing wave of velocity fluctuation and a standing wave of pressure fluctuation when N = 5 and one end is fixed.

In FIGs. 4A to 4D and 5A to 5C, a solid line represents a velocity distribution and a dotted line represents a pressure distribution. Standing wave are actually compressional waves (longitudinal waves), but are commonly expressed as presented in FIGs. 4A to 4D and 5A to 5C. A solid line represents a velocity standing wave and a dotted line represents a pressure standing wave. For example, as can be seen from FIG. 4A in which N = 1 and one end is fixed, an amplitude of the velocity distribution is zero at a closed end and the amplitude reaches the maximum at an opened end, which is intuitively understandable. Assuming that a length between both ends of the liquid-column-resonance liquid chamber in a longitudinal direction is L and a wavelength at which liquid column resonance of liquid occurs is $\lambda$, the standing wave is most efficiently generated when the integer N is from 1 through 5. A standing wave pattern varies depending on whether each end is opened or closed. Therefore, standing wave patterns under various opening/closing conditions are also described in the drawings. As described below, conditions of the ends are determined depending on states of openings of the discharging holes and states of openings at a supplying side.

Note that, in the acoustics, an opened end refers to an end at which moving velocity of a medium reaches the local maximum, but, to the contrary, pressure of the medium is zero. Conversely, a closed end refers to an end at which moving velocity of a medium (liquid) is zero in a longitudinal direction, but, to the contrary, pressure of the medium reaches the local maximum. The closed end is considered as an acoustically hard wall and reflects a wave. When an end is ideally perfectly closed or opened, resonance standing waves as presented in FIGs. 4A to 4D and 5A to 5C are formed by superposition of waves. However, standing wave patterns vary depending on the number of the discharging holes and positions at which the discharging holes are opened. Therefore, a resonance frequency appears at a position shifted from a position determined according to the Expression 3. In this case, conditions under which liquid droplets are stably formed can be created by appropriately adjusting the driving frequency. For example, when the sound velocity c of the liquid is 1,200 m/s, the length L of the liquid-column-resonance liquid chamber is 1.85 mm, and a resonance mode, in which both ends are completely equivalent to fixed ends due to the presence of walls on the both ends and N = 2, is used, the most efficient resonance frequency is calculated as 324 kHz from the Expression 2. In another example, when the sound velocity c of the liquid is 1,200 m/s and the length L of the liquid-column-resonance liquid chamber is 1.85 mm, these conditions are the same as above, and a resonance mode, in which both ends are equivalent to fixed ends due to the presence of walls at the both ends and N = 4, is used, the most efficient resonance frequency is calculated as 648 kHz from the Expression 2. Thus, a higher-order resonance can be utilized even in a liquid-column-resonance liquid chamber having the same configuration.

In order to increase the frequency, the liquid-column-resonance liquid chamber of the liquid droplet forming unit 11 presented in FIG. 1 preferably has both ends which are equivalent to a closed end or are considered as an acoustically soft wall due to influence from openings of the discharging holes. However, the both ends may be free. The influence from openings of the discharging holes means decreased acoustic impedance and, in particular, an increased compliance component. Therefore, the configuration, in which walls are formed at both ends of the liquid-column-resonance liquid chamber in a longitudinal direction, as presented in FIGs. 4B and 5A, is preferable because it is possible to use both of a resonance mode in which both ends are fixed and a resonance mode in which one of ends is free, that is, an end at a discharging hole side is considered to be opened.

The number of openings of the discharging holes, positions at which the openings are disposed, and cross-sectional shapes of the discharging holes are also factors which determine the driving frequency. The driving frequency may be appropriately determined based on these factors. For example, when the number of the discharging holes is increased, the liquid-column-resonance liquid chamber gradually becomes free at an end which has been fixed. As a result, a resonance standing wave which is approximately the same as a standing wave at the opened end is generated and the driving frequency is increased. Further, the end which has been fixed becomes free starting from a position at which an opening of the discharging hole that is the closest to the liquid supplying path is disposed. As a result, a cross-sectional shape of the discharging hole is changed to a round shape or a volume of the discharging hole is varied depending on a thickness of the frame, so that an actual standing wave has a shorter wavelength and a higher frequency than the driving frequency. When a voltage is applied to the vibration generating unit at the driving frequency determined as described above, the vibration generating unit deforms and the resonance standing wave is generated most efficiently at the driving frequency. The liquid column resonance standing wave is also generated at a frequency adjacent to the driving frequency at which the resonance standing wave is generated most efficiently. That is, assuming that a length between both ends of the liquid-column-resonance liquid chamber in a longitudinal direction is L and a distance to a discharging hole that is the closest to an end at a liquid supplying side is Le, the driving frequency f is determined according to Expression 4 and Expression 5 below using both of the lengths L and Le. A driving waveform having, as a main component, the driving frequency f can be used to vibrate the vibration generating unit and to induce the liquid column resonance to thereby discharge the liquid droplets from the discharging holes for formation of liquid droplets.

$$N \times c / (4L) \leqq f \leqq N \times c / (4Le) \cdots \text{(Expression 4)}$$

$$N \times c / (4L) \leqq f \leqq (N + 1) \times c / (4Le) \cdots \text{(Expression 5)}$$

In the Expressions 4 and 5, L denotes a length of the liquid-column-resonance liquid chamber in a longitudinal direction; Le denotes a distance from an end at a liquid supplying path side to a center of a discharging hole that is the closest to the end; c denotes velocity of an acoustic wave of a liquid; and N denotes a natural number.

Note that, a ratio (L/Le) of the length L between both ends of the liquid-column-resonance liquid chamber in a longitudinal direction to the distance Le to the discharging hole that is the closest to the end at the liquid supplying side preferably satisfies Expression 6 below.

$$Le/L > 0.6 \cdots \text{(Expression 6)}$$

Based on the principle of the liquid column resonance phenomenon described above, a liquid-column resonance pressure standing-wave is formed in the liquid-column-resonance liquid chamber 18 presented in FIG. 1, and continuous discharging is performed from the discharging holes 19 disposed in a portion of the liquid-column-resonance liquid chamber 18, to thereby form liquid droplets. Note that, the discharging hole 19 is preferably disposed at a position at which pressure of the standing wave varies to the greatest extent from the viewpoints of high efficiency of forming liquid droplets and driving at a lower voltage. One liquid-column-resonance liquid chamber 18 may include one discharging hole 19, but preferably includes two or more (a plurality of) discharging holes from the viewpoint of productivity. Specifically, the number of discharging holes is preferably 2 or more but 100 or less. When the number of discharging holes is 2 or more, productivity can be improved. When the number of discharging holes is 100 or less, a voltage to be applied to the vibration generating unit 20 may be set at a low level in order to form desired liquid droplets from the discharging holes 19. As a result, a piezoelectric material can stably behave as the vibration generating unit 20.

When the plurality of the discharging holes 19 are disposed, a pitch between the discharging holes (the shortest distance between centers of discharging holes adjacent to each other) is preferably 20 $\mu$m or longer but equal to or shorter than the length of the liquid-column-resonance liquid chamber. When the pitch between the discharging holes is 20 $\mu$m or more, it is possible to decrease the possibility that liquid droplets, which are discharged from discharging holes adjacent to each other, collide with each other to form a larger droplet. As a result, particles having a good particle diameter distribution may be obtained.

Next, a liquid column resonance phenomenon which occurs in the liquid-column-resonance liquid chamber of a liquid-droplet discharging head of the liquid droplet forming unit will be described with reference to FIGs. 6A to 6E. Note that, in FIGs. 6A to 6E, a solid line drawn in the liquid-column-resonance liquid chamber represents a velocity distribution plotting velocity at arbitrary measuring positions between an end at the fixed end side and an end at the common liquid supplying path side in the liquid-column-resonance liquid chamber. A direction from the common liquid supplying path to the liquid-column-resonance liquid chamber is assumed as plus (+), and the opposite direction is assumed as minus (-). A dotted line drawn in the liquid-column-resonance liquid chamber represents a pressure distribution plotting pressure at arbitrary measuring positions between an end at the fixed end side and an end at the common liquid supplying path side in the liquid-column-resonance liquid chamber. A positive pressure relative to atmospheric pressure is assumed as plus (+), and a negative pressure is assumed as minus (-). In the case of the positive pressure, pressure is applied in a downward direction in the drawings. In the case of the negative pressure, pressure is applied in an upward direction in the drawings. In FIGs. 6A to 6E, as described above, the end at the liquid supplying path side is free, and the height of the frame serving as the fixed end (height h1 in FIG. 1) is about 2 times or more as high as the height of an opening at which the liquid supplying path 17a is in communication with the liquid-column-resonance liquid chamber 18 (height h2 in FIG. 1). Therefore, FIGs. 6A to 6E represent temporal changes of a velocity distribution and a pressure distribution under an approximate condition in which the liquid-column-resonance liquid chamber 18 are approximately fixed at both ends. In FIGs. 6A to 6E, a solid line represents a velocity distribution and a dotted line represents a pressure distribution.

A schematic view presenting one example of liquid column resonance phenomenon that occurs in a liquid column resonance flow path of a liquid droplet forming unit. FIG. 6A presents a pressure waveform and a velocity waveform in the liquid-column-resonance liquid chamber 18 at a time when liquid droplets are discharged. In FIG. 6B, meniscus pressure is increased again after the liquid droplets are discharged and immediately then the liquid is drawn. As presented in FIGs. 6A and 6B, pressure in a flow path, on which the discharging holes 19 are disposed, in the liquid-column-resonance liquid chamber 18 is the local maximum. Then, as presented in FIG. 6C, positive pressure adjacent to the discharging holes 19 is decreased and shifted to a negative pressure side. Thus, the liquid droplets 21 are discharged.

Then, as presented in FIG. 6D, the pressure adjacent to the discharging holes 19 is the local minimum. From this time point, the liquid-column-resonance liquid chamber 18 starts to be filled with the liquid 14. Then, as presented in FIG. 6E, negative pressure adjacent to the discharging holes 19 is decreased and shifted to a positive pressure side. At this time point, the liquid chamber is completely filled with the liquid (particle composition liquid) 14. Then, as presented in FIG. 6A, positive pressure in a liquid-droplet discharging region of the liquid-column-resonance liquid chamber 18 is the local maximum again to discharge the liquid droplets 21 from the discharging holes 19. Thus, the liquid column resonance standing wave is generated in the liquid-column-resonance liquid chamber by the vibration generating unit driven at a high frequency. The discharging holes 19 are disposed in the liquid-droplet discharging region corresponding to the anti-nodes of the liquid column resonance standing wave at which pressure varies to the greatest extent. Therefore, the liquid droplets 21 are continuously discharged from the discharging holes 19 in synchronized with an appearance cycle of the anti-nodes.

One exemplary aspect where liquid droplets are actually discharged based on the liquid column resonance phenomenon will now be described. FIG. 7 is an image presenting exemplary actual liquid droplets discharged by a liquid droplet forming unit. In this example, liquid droplets were discharged under the below-described conditions: the length L between both ends of the liquid-column-resonance liquid chamber 18 in a longitudinal direction in FIG. 1 was 1.85 mm, a resonance mode was N = 2, the first to fourth discharging holes were disposed at positions corresponding to anti-nodes of a pressure standing wave in the resonance mode of N = 2, and the driving frequency was a sine wave at 340 kHz. FIG. 7 is a photograph of the thus-discharged liquid droplets, and the photograph was taken by laser shadowgraphy. As can be seen from FIG. 7, the liquid droplets which are very uniform in diameter and substantially uniform in velocity are successfully discharged.

FIG. 8 is a graph presenting dependency of a liquid droplet-discharging velocity on a driving frequency when driven by a sine wave having the same amplitude of 290 kHz or more but 395 kHz or less as the driving frequency. As can be seen from FIG. 8, a discharging velocity of liquid droplets from each of the first to fourth nozzles is uniform and is the maximum discharging velocity adjacent to the driving frequency of about 340 kHz. It can be seen from this result that uniform discharging is achieved at a position corresponding to an anti-node of the liquid column resonance standing wave at 340 kHz which is the second mode of a liquid column resonance frequency. It can also be seed from the results in FIG. 8 that a frequency characteristic of liquid column resonance standing waves characteristic of the liquid column resonance occurs in the liquid-column-resonance liquid chamber. The frequency characteristic is that liquid droplets are not discharged between a liquid droplet-discharging velocity peak at 130 kHz, which is the first mode, and a liquid droplet-discharging velocity peak at 340 kHz, which is the second mode.

FIG. 9 is a schematic view presenting one example of a particle production apparatus. A particle production apparatus 1 presented in FIG. 9 mainly includes a liquid droplet forming unit 2, a drying·collection unit 60, a conveyance gas flow discharging port 65, and a particle storage section 63. The liquid droplet forming unit 2 is coupled to a raw material housing container 13 configured to house a liquid 14 through a liquid supplying pipe 16 and a liquid returning pipe 22. The liquid supplying pipe 16 is coupled to a liquid circulating pump 15. The liquid circulating pump 15 is configured to supply the liquid 14 housed in the raw material housing container 13 to the liquid droplet forming unit 2 through the liquid supplying pipe 16, and to feed the liquid 14 in the liquid supplying pipe 16 under pressure to return to the raw material housing container 13 through a liquid returning pipe 22. This configuration makes it possible to supply the liquid 14 to the liquid droplet forming unit 2 at all times. The liquid supplying pipe 16 is provided with a pressure gauge P1 and the drying·collection unit 60 is provided with a pressure gauge P2. The pressure at which the liquid is fed to the liquid droplet forming unit 2 and the pressure within the drying·collection unit are controlled by pressure gauges P1 and P2. When a value of pressure measured at P1 is larger than a value of pressure measured at P2, there is a risk that the liquid 14 is oozed from the discharging hole. When a value of pressure measured at P1 is smaller than a value of pressure measured at P2, there is a risk that a gas enters the liquid droplet forming unit 2 to stop discharging. Therefore, it is preferable that a value of pressure measured at P1 and a value of pressure measured at P2 be substantially the same.

Within a chamber 61, a downward gas flow (conveyance gas flow) 101 generated from a conveyance gas flow introducing port 64 is formed. A liquid droplet 21 discharged from the liquid droplet forming unit 2 is conveyed downward not only through gravity but also through the conveyance gas flow 101, passes through the conveyance gas flow discharging port 65, is collected by a collecting unit 62, and is stored in the particle storage section 63.

When discharged liquid droplets contact with each other before they are dried, the liquid droplets are unified to form a single particle (hereinafter, this phenomenon may be referred to as "cohesion"). In order to obtain particles having a uniform particle size distribution, it is necessary to maintain a distance between the discharged liquid droplets. Although the discharged liquid droplet travels at a certain initial velocity, the velocity is decreased soon due to air resistance. The liquid droplet decreased in the velocity is caught up with by a liquid droplet subsequently discharged, which leads to cohesion. This phenomenon occurs regularly. Therefore, when particles are collected, the particle size distribution considerably becomes worsened. In order to prevent cohesion, it is preferable to dry (solidify) and convey liquid droplets, while the velocity of the liquid droplet is prevented from being decreased and the liquid droplets do not contact with each other to prevent cohesion by the conveyance gas flow 101. Finally, it is preferable to convey the particles to the collecting unit.

As presented in FIG. 9, a part of the conveyance gas flow 101 as the first gas flow is provided near the liquid droplet forming unit in the same direction as the direction in which the liquid droplet is discharged. As a result, the velocity of the liquid droplet immediately after the liquid droplet is discharged is prevented from being decreased, which makes it possible to prevent cohesion. FIG. 10 is a schematic view presenting one exemplary gas flow path. The gas flow in the gas flow path 12 may be orientated in a direction transverse to the liquid-droplet discharging direction, as presented in FIG. 10. Alternatively, although not presented, the gas flow may be oriented at a certain angle, and the certain angle is preferably such an angle that the liquid droplets are spaced from each other by the liquid droplet forming unit. As presented in FIG. 10, when a cohesion preventing gas flow is provided from the direction transverse to the direction in which the liquid droplet is discharged, the cohesion preventing gas flow is preferably orientated in a direction in which trajectories of the liquid droplets do not overlap with each other when the liquid droplets are conveyed from the discharging holes by the cohesion preventing gas flow.

After cohesion is prevented by the first gas flow as described above, the dried particles may be conveyed to the collecting unit by the second gas flow.

The velocity of the first gas flow is preferably equal to or higher than the velocity of the liquid droplet to be discharged. When the velocity of the cohesion preventing gas flow is lower than the velocity of the liquid droplet to be discharged, it may be difficult to exhibit a function of preventing liquid droplets from contacting with each other, which is an original purpose of the cohesion preventing gas flow.

As a property of the first gas flow, such a condition that the liquid droplets do not cohere can be added, and the property of the first gas flow may be different from that of the second gas flow. Moreover, such a chemical substance that facilitates drying of the surfaces of the particles may be mixed with or added to the cohesion preventing gas flow, in expectation of physical action.

A state of the conveyance gas flow 101 is not particularly limited to a state of the gas flow. The conveyance gas flow 101 may be a laminar flow, a rotational flow, or a turbulent flow. Kinds of gases constituting the conveyance gas flow 101 are not particularly limited and may be appropriately selected depending on the intended purpose. For example, air may be used, or an incombustible gas such as nitrogen may be used. A temperature of the conveyance gas flow 101 may be appropriately adjusted. Preferably, the temperature thereof is not changed at the time of production. A unit configured to change a gas flow condition of the conveyance gas flow 101 may be included within the chamber 61. The conveyance gas flow 101 may be used not only for prevention of cohesion of the liquid droplets 21 but also for prevention of attachment to the chamber 61.

When an amount of the residual solvent contained in the particles obtained by the particle collecting unit presented in FIG. 9 is large, the secondary drying is preferably performed if necessary in order to decrease the residual solvent. As the secondary drying, generally known drying units such as fluidized bed drying and vacuum drying can be used.

(Pharmaceutical composition and method of use thereof)

The pharmaceutical composition of the present invention contains the functional particles of the present invention, and typically contains instantly soluble particles as an active ingredient. The pharmaceutical composition may further contain other ingredients, if necessary.

The pharmaceutical composition of the present invention can be suitably produced by the below-described method of the present invention for producing a pharmaceutical composition.

In the present invention, the "pharmaceutical composition" refers to a composition containing the functional particles of the present invention, typically instantly soluble particles, each containing a kinase inhibitor that is a poorly water-soluble compound, where when added to water or physiological saline, the functional particles dissolve in the water or physiological saline to be able to obtain a solution or dispersion liquid of the kinase inhibitor, and the composition is used particularly for the treatment of injuries and diseases.

As a result of diligent studies conducted by the present inventors, they have found that by containing the functional particles, typically instantly soluble particles, in a pharmaceutical composition, it rapidly dissolves in water or physiological saline after formation into particles and the kinase inhibitor retains its physiological activity.

<Functional particles>

The functional particles in the pharmaceutical composition of the present invention each contain a water-soluble base material a kinase inhibitor that is a poorly water-soluble compound, and contain other ingredients, if necessary.

-Water-soluble base material-

The water-soluble base material is similar to that described in the section -Water-soluble base material- of the above (Functional particles).

-Kinase inhibitor-

The pharmaceutical composition of the present invention contains a kinase inhibitor as an active ingredient. The kinase inhibitor that can be contained in the pharmaceutical composition of the present invention is similar to that described in the section -Poorly water-soluble compound- of the above (Functional particles).

The volume average particle diameter (Dv) and the R.S.F of the functional particles in the pharmaceutical composition are similar to the volume average particle diameter (Dv) and the R.S.F of the functional particles described in the above section (Functional particles).

-Other ingredients-

The other ingredients in the functional particles are similar to those described in the section -Other ingredients- of the above (Functional particles).

The other ingredients which the pharmaceutical composition may further contain in addition to the functional particles are not particularly limited and may be appropriately selected depending on the intended purpose. For example, the other ingredients may be appropriately selected from those described in the section -Other ingredients- of the above (Functional particles). The amount thereof in the pharmaceutical composition may be appropriately selected.

The pharmaceutical composition has instant solubility and can be prepared at the time of use. In the present invention, that the pharmaceutical composition "has instant solubility" means that the pharmaceutical composition dissolves in water or physiological saline such rapidly that the pharmaceutical composition can be administered by dissolving the pharmaceutical composition in the physiological saline at the time of administration.

A method usable for confirming whether the pharmaceutical composition has instant solubility is, for example, the method described in the above section of the functional particles. Another confirmation method is, for example, where the presence of instant solubility is determined when an active ingredient in such an amount as to give a pharmaceutically effective concentration is added under dissolution conditions used for the elution test stipulated in the Japanese Pharmacopeia, and elution equal to or more than a predetermined amount (e.g., 85% of the amount of the active ingredient added) within a predetermined period of time (e.g., within 30 minutes) is found. In such a method, examples of the predetermined period of time include, but are not limited to, 30 minutes, 25 minutes, 20 minutes, 15 minutes, 10 minutes, 5 minutes, 4 minutes, 3 minutes, 2 minutes, 1 minute, 45 seconds, 30 seconds, 25 seconds, 20 seconds, 15 seconds, 10 seconds, and 5 seconds. Examples of the predetermined amount include, but are not limited to, 85%, 90%, 95%, 96%, 97%, 98%, 99%, and 100% of the amount of the active ingredient added. The "pharmaceutically effective concentration" is different depending on the active ingredient to be added and a route of administration thereof. Persons skilled in the art, however, could calculate the pharmaceutically effective concentration of a specific active ingredient to be administered via a specific route of administration.

The pharmaceutical composition of the present invention is typically provided in the form of powder. However, since the pharmaceutical composition of the present invention contains the functional particles, the pharmaceutical composition can also be dissolved in water or physiological saline immediately before administration. In particular, the pharmaceutical compound that can be contained in the pharmaceutical composition of the present invention is the poorly water-soluble compound, and thus the pharmaceutical composition is preferably dissolved in water or physiological saline for use and more preferably dissolved in water or physiological saline at the time of use. When the pharmaceutical composition is prepared at the time of use, a solution of the pharmaceutical composition dissolved in water or physiological saline can be suitably administered as, for example, an oral liquid preparation to be orally taken, an injection to be injected into, for example, a blood vessel, or an inhalant to be orally or nasally administered after atomization.

- Diseases-

The target disease for which the pharmaceutical composition is to be used is not particularly limited and may be appropriately selected from diseases caused by activation of a kinase depending on, for example, the kind of the kinase inhibitor.

Examples of the diseases caused by activation of a kinase include pulmonary fibrosis, non-small-cell lung cancer, pancreatic cancer, pancreatic neuroendocrine tumor, gastrointestinal stromal tumor, renal cell cancer, hepatocellular cancer, thyroid cancer, medullary thyroid cancer, breast cancer, colon or rectum cancer, malignant soft tissue tumor, acute myelogenous leukemia, chronic myelogenous leukemia, acute lymphocytic leukemia, chronic lymphocytic leukemia, chronic eosinophilic leukemia, hypereosinophilic syndrome, and mantle-cell lymphoma.

The present invention includes a treatment method of the target disease that includes administering the pharmaceutical composition of the present invention to a subject that suffers from the target disease.

-Method of administration-

When administering the pharmaceutical composition of the present invention, administration forms, administration routes, doses, interval between administrations, timings of administration, periods of administration, and subjects of administration are not particularly limited and may be appropriately selected depending on the intended purpose.

Examples of the administration forms of the pharmaceutical composition of the present invention include oral preparations, injections (including those that are dissolved at the time of use), and external preparations. The pharmaceutical composition of the present invention is typically provided in the form of powder. However, the pharmaceutical composition may be, for example, tableted or dissolved in water or physiological saline before administration (including immediately before administration). Thus, the pharmaceutical composition may be prepared in any form of a solid preparation, a semi-solid preparation, and a liquid preparation.

Examples of the oral preparations include tablets (including sugar-coated tablets, sublingual tablets, and oral tablets), capsules, granules, powders, fine granules, syrup (including dry syrup), enteric coated preparations, sustained-release capsules, cashew (including wafer capsules), chewing tablets, drops, pills, liquid preparations for internal use, confectionery tablets (e.g., troches and candy), sustained-release tablets, and sustained-release granules.

Examples of the external preparations include sprinkling powders, lotion, ointment/cream, shampoo, spray, liquid preparations for external use (including liniments), tapes (including plasters), aerosol, ear drops, eye drops, eye ointment, nasal drops (including nasal spray), inhalants (including inhalation anesthetics and spray for inhalation), spin caps, gargle preparations, suppositories, inserts, enemas, and jelly.

The administration route is not particularly limited and may be appropriately selected depending on the intended purpose. Examples thereof include topical administration, enteral administration, and parenteral administration.

Examples of the topical administration include transairway administration (intratracheal administration), enema administration, administration onto the skin, eye drops onto the conjunctiva, ear drops, transnasal administration, and intravaginal administration.

Examples of the enteral administration include oral administration, transluminal administration, and enema administration.

Examples of the parenteral administration include: parenteral administration with a syringe or an infusion pump, such as transvenous administration, transarterial administration, intramuscular administration, intracardiac administration, subcutaneous administration, intraosseous administration, intradermal administration, subarachnoid (cavity) administration, intraperitoneal administration, and intravesical administration; percutaneous administration; transmucosal administration; and inhalation administration.

Since the pharmaceutical composition of the present invention enable a poorly water-soluble kinase inhibitor to rapidly dissolve in water or physiological saline, the pharmaceutical composition can be used as an inhalation liquid preparation for topical administrations such as transairway administration, via which when the kinase inhibitor is a poorly water-soluble compound, it was hitherto necessary to administer it as an inhalation powder or inhalation aerosol. Therefore, particularly preferably, the pharmaceutical composition of the present invention is administered to a subject in the form of an inhalation liquid preparation using, for example, a nebulizer.

The dose is not particularly limited and may be appropriately selected depending on the intended purpose. As presented in the below-described test examples, it may be possible for the pharmaceutical composition of the present invention to achieve desired physiological activity at a less dose thereof than the conventionally employed dose.

The interval between administrations is not particularly limited and may be appropriately selected depending on the intended purpose.

The timing of administration is not particularly limited and may be appropriately selected depending on the intended purpose. It may be administered before the onset of a disease for the purpose of prevention. It may also be administered after the onset of a disease for the purpose of ameliorating symptoms or delay progression of symptoms.

In the present invention, the "treatment" includes prevention of the onset of a disease, suppression of progression of symptoms, and amelioration of symptoms.

The period of administration is not particularly limited and may be appropriately selected depending on the intended purpose.

The subject of administration is not particularly limited and may be appropriately selected depending on the intended purpose. Examples thereof include humans; mammals such as non-human primates (e.g., monkeys), pigs, cows, sheep, goats, dogs, cats, mice, and rats; and avian such as birds.

(Method for producing pharmaceutical composition and apparatus for producing pharmaceutical composition)

A method of the present invention for producing a pharmaceutical composition includes: a liquid droplet forming step of discharging a liquid containing a rapidly water-soluble compound and a kinase inhibitor, which is a poorly water-soluble compound, from a discharging hole to form liquid droplets; and a particle forming step of solidifying the liquid droplets to form particles. The method of the present invention further includes other steps, if necessary.

The method of the present invention for producing a pharmaceutical composition can be performed similar to the method for producing functional particles described in the above section (Method for producing functional particles and apparatus for producing functional particles).

An apparatus of the present invention for producing a pharmaceutical composition includes: a liquid droplet forming unit configured to discharge a liquid containing a rapidly water-soluble compound and a kinase inhibitor, which is a poorly water-soluble compound, from a discharging hole to form liquid droplets; and a particle forming unit configured to solidify the liquid droplets to form particles. The apparatus of the present invention further includes a particle collecting unit and other units, if necessary.

The apparatus of the present invention for producing a pharmaceutical composition is similar to the apparatus for producing functional particles described in the above section (Method for producing functional particles and apparatus for producing functional particles).

Examples

The present invention will now be described by way of Examples and Test Examples. The present invention should not be construed as being limited to these Examples and Test Examples in any way.

(Example 1)

<Preparation of liquid A>

Tyrphostin (obtained from Tokyo Chemical Industry Co., Ltd.) (2 parts by mass) and lactose monohydrate (obtained from Tokyo Chemical Industry Co., Ltd.) (8 parts by mass) were added to a mixture solvent of water (700 parts by mass) and methanol (300 parts by mass), followed by dissolving the resultant to obtain liquid A.

<Production of instantly soluble particle 1>

A liquid column resonance droplet-discharging apparatus (obtained from Ricoh Company, Ltd.) of FIG. 1, in which the number of openings of the discharging holes was set to one per one liquid-column-resonance liquid chamber, was used to discharge the obtained liquid A from the discharging hole to form liquid droplets. Then, the apparatus of FIG. 9 was used to dry the liquid droplets to obtain instantly soluble particle 1. Here, particle formulation conditions are as follows.

-Particle formulation conditions-
--Liquid column resonance conditions--
• Resonance mode (N): 2
• Length (L) between both ends in longitudinal direction of liquid-column-resonance liquid chamber: 1.8 mm
• Height (h1) of end of frame at common liquid supplying path side of liquid-column-resonance liquid chamber: 80 μm
• Height (h2) of communication hole of liquid-column-resonance liquid chamber: 40 μm
--Liquid droplet formation unit--
• Shape of discharging hole: perfect circle
• Diameter of discharging hole: 8.0 μm
• Number of openings of discharging holes: 1 (per one liquid-column-resonance liquid chamber)
• Number of liquid-column-resonance liquid chambers : 384 chambers
• Applied voltage: 12.0 V
• Driving frequency: 310 kHz
--Particle formulation unit--
• Conveyance gas flow: dry nitrogen
• Temperature of conveyance gas flow: 40°C
• Flow rate of conveyance gas flow: 100 L/min

(Example 2)

<Preparation of liquid B>

Liquid B was prepared in the same manner as in Example 1 except that the formulation in Example 1 was changed to the formulation presented in Table 1.

<Production of instantly soluble particles 2>

Instantly soluble particles 2 were obtained in the same manner as in Example 1 except that a spray dryer (apparatus name: GS310, obtained from Yamato Scientific Co., Ltd.) was used to form the obtained liquid B into liquid droplets. Here, particle formulation conditions are as follows.

- Particle formulation conditions-
-- Liquid droplet formation unit--
• Shape of discharging hole: perfect circle
• Diameter of discharging hole: 0.5 mm
• Number of openings of discharging holes: 1
• Discharging air pressure: 0.1 MPa
-- Particle formulation unit--
• Conveyance gas flow: dry nitrogen
• Temperature of conveyance gas flow: 75°C
• Flow rate of conveyance gas flow: 500 L/min

(Examples 3 to 6)

Liquids C to F were prepared and instantly soluble particles 3 to 6 were obtained in the same manner as in Example 1 except that the formulation in Example 1 was changed to the formulations presented in Table 1.

(Example 7)

<Preparation of liquid G>

Liquid G was prepared in the same manner as in Example 1 except that the formulation in Example 1 was changed to the formulation presented in Table 1.

<Production of instantly soluble particles 7>

Instantly soluble particles 7 was obtained in the same manner as in Example 1 except that the prepared liquid G was formed into liquid droplets using a particle production apparatus provided with a film vibration-type nozzle (obtained from Optnics Precision CO., LTD.). Here, particle formulation conditions of the particles are as follows.

- Particle formulation conditions-
-- Liquid droplet formation unit--
• Shape of discharging hole: perfect circle
• Diameter of discharging hole: 10 μm
• Applied voltage: 20.0 V
• Driving frequency: 100 KHz
-- Particle formulation unit--
• Temperature of dry air: 40°C
• Flow rate of dry air: 500 L/min

(Example 8)

Liquid H was prepared and Instantly soluble particles 8 was obtained in the same manner as in Example 2 except that the formulation in Example 2 was changed to the formulation presented in Table 1.

(Comparative Examples 1 and 2)

Liquids I and J were prepared and Instantly soluble particles 9 and 10 were obtained in the same manner as in Example 1 except that the formulation in Example 1 was changed to the formulation presented in Table 1.

Table 1

| | | Poorly water-soluble compound (Pharmaceutical compound) | | Water-soluble base material (Rapidly water-soluble compound) | | Solvent 1 | | Solvent 2 | |
|---|---|---|---|---|---|---|---|---|---|
| | | Name | Amount (parts by mass) | Name | Amount (parts by mass) | Name | Amount (parts by mass) | Name | Amount (parts by mass) |
| Ex. | 1 | Tyrphostin | 2 | Lactose monohydrate | 8 | Water | 700 | Methanol | 300 |
| | 2 | Tyrphostin | 2 | Lactose monohydrate | 8 | Water | 350 | Methanol | 150 |
| | 3 | Tyrphostin | 7 | Lactose monohydrate | 3 | Water | 700 | Methanol | 300 |
| | 4 | Tyrphostin | 2 | Mannitol | 8 | Water | 350 | Methanol | 150 |
| | 5 | Gefitinib | 5 | Glucose | 5 | Water | 700 | Ethanol | 300 |
| | 6 | Gefitinib | 7 | Lactose monohydrate | 3 | Water | 700 | Ethanol | 300 |
| | 7 | Gefitinib | 2 | Mannitol | 8 | Water | 700 | Ethanol | 300 |
| | 8 | Cyclosporine | 2 | Glucose | 8 | Water | 230 | Ethanol | 100 |
| Comp. Ex. | 1 | Tyrphostin | 2 | HPC-L | 8 | Ethanol | 700 | - | - |
| | 2 | Tyrphostin | 2 | Eudragit RL | 8 | Methanol | 700 | - | - |

Next, particles 1 to 10 obtained in Examples 1 to 8 and Comparative Examples 1 and 2 were measured and evaluated for "amount of poorly water-soluble compound in particles" and "solubility" in the following manners. Results are presented in Table 2.

<Amount of poorly water-soluble compound in particles>

An amount of the poorly water-soluble compound in each of the produced particles 1 to 10 was measured through liquid chromatograph (detector: mass spectrometer).
Results are presented in Table 2.

<Evaluation of solubility>

The formed particle was weighed and added to physiological saline (10 g) so that the concentration of the drug would be 1% by mass. After the addition, a dissolution state when the resultant was shaken by hand at two times/sec was evaluated based on the following evaluation criteria. The time of the handshake was performed with three levels (10 seconds, 20 seconds, and 30 seconds), and the dissolution state at that time was confirmed. Considering practical use in clinical sites, A, B, and C were considered acceptable. Results are presented in Table 2. Note that, the phrase "the particles were completely dissolved" means that the particles can be visually confirmed that there is no remaining particle.

[Evaluation criteria]

A: Completely dissolved within 10 seconds.
B: Completely dissolved within 20 seconds.
C: Completely dissolved within 30 seconds.
D: Longer than 30 seconds.

Table 2

| | | Particle No. | Particle formation conditions | Evaluation Results: Amount of poorly water-soluble compound in particles (% by mass) | Evaluation Results: Solubility |
|---|---|---|---|---|---|
| Ex. | 1 | 1 | Liquid column resonance | 20 | A |
| | 2 | 2 | Two-fluid spray | 20 | B |
| | 3 | 3 | Liquid column resonance | 70 | C |
| | 4 | 4 | Liquid column resonance | 20 | A |
| | 5 | 5 | Liquid column resonance | 50 | B |
| | 6 | 6 | Liquid column resonance | 70 | C |
| | 7 | 7 | Film vibration | 20 | B |
| | 8 | 8 | Two-fluid spray | 20 | B |
| Comp. Ex. | 1 | 9 | Liquid column resonance | 20 | D |
| | 2 | 10 | Liquid column resonance | 20 | D |

In Examples 1 to 8, all of the powdery pharmaceutical preparations were completely dissolved within 30 seconds to obtain uniformly transparent solutions. Meanwhile, in Comparative Examples 1 and 2, the aqueous solutions were found to have cloudiness as a whole even after 30 seconds.

Nintedanib ethanesulfonate, one of the tyrosine kinase inhibitors, was used as one example of pharmaceutical compounds. Nintedanib ethanesulfonate is known as an active ingredient of a therapeutic drug for idiopathic pulmonary fibrosis. A pharmaceutical composition containing nintedanib ethanesulfonate was tested for, for example, solubility and physiological activity in the following manners.

(Test Example 1: Solubility)

<Production of pharmaceutical composition (i)>

Pharmaceutical composition (i) as instantly soluble particles were obtained in the same manner as in Example 1 except that the liquid A was changed to liquid (i) having the following formulation.

-Liquid (i)-

- Nintedanib ethanesulfonate (obtained from LC laboratories): 0.1 parts by mass (0.083 parts by mass as nintedanib)
- Lactose monohydrate (obtained from Tokyo Chemical Industry Co., Ltd.): 9.9 parts by mass
- Solvent: 1,000 parts by mass (solvent mixture of 700 parts by mass of water and 300 parts by mass of methanol)

The amount of nintedanib ethanesulfonate in the obtained pharmaceutical composition (i) was measured in the same manner as in the method described in the section <Amount of poorly water-soluble compound in particles> and was found to be 0.1% by mass (0.083% by mass as nintedanib).

<Dissolution test>

The pharmaceutical composition (i) or the nintedanib ethanesulfonate (obtained from LC laboratories) was added to 100 mL of physiological saline so that the amount of nintedanib would be 6 mg/mL. The resultant mixture was subjected to a dissolution test at a liquid temperature of 20°C under stirring with a stirrer.
FIG. 11 is a graph presenting the concentrations of nintedanib in the solutions measured over time.
As presented in FIG. 11, when the nintedanib sulfonate was dissolved, solubility decreased over time and sedimentation of insoluble matter was observed. Meanwhile, the pharmaceutical composition (i) was dissolved, it rapidly dissolved and also solubility increased over time. At a dissolution time of 30 minutes, the pharmaceutical composition (i) was found to 70 or greater times increase in solubility as compared with the nintedanib ethanesulfonate.

(Test Example 2: Animal experiments)

As one example of confirmation that the pharmaceutical composition of the present invention has physiological activity, physiological activity and other properties of the pharmaceutical composition (i) produced in the Test Example 1 were verified using bleomycin pulmonary fibrosis model mice.

<Verification method>

«1. Settings by group»

(1) Control group

This is a group that received no bleomycin and received physiological saline. Regarding the administration route, there was only the transairway administration group.

(2) Treatment groups

These are groups that each received the pharmaceutical composition (i) at 20 pg/mouse, 60 pg/mouse, or 120 pg/mouse as the amount of nintedanib 48 hours before administration of bleomycin (hereinafter may be referred to as "Day -2"). Regarding the administration route, there were two kinds of groups: the transairway administration group (hereinafter may be referred to as a "transairway treatment group); and the transabdominal group (hereinafter may be referred to as a "transabdominal treatment group").

(3) Non-treatment group

This is a group that received only the bleomycin.

«2. Evaluation items»

(1) Observation of systemic conditions

Every other day or three times in a week, the mice were measured for body weight and observed for appearance. The mice that had been found to reduce in body weight by 20% or higher were euthanized.

(2) Respiratory function test

A respiratory function measuring device for small animals (Flexivent, obtained from emkaTECHNOLOGIES) was used for evaluation at the 21$^{st}$ day when pulmonary fibrosis was completed (the 21$^{st}$ day from the 0 day as the administration day of bleomycin; hereinafter may be referred to as "Day 21").

«3. Mice, reagents, etc.»

(1) Mice
8 to 10-week-old Balb/c female mice (the body weight of which was assumed to be from 18 to 22 g) (6 to 7-week-old mice were purchased and conditions for 1 to 2 weeks before use)
(2) Drug: Pharmaceutical composition (i) produced in Test Example 1
(3) Bleomycin (EMD-millipore Cat# 203401 Bleomycine Sulfate 10 mg)
(4) Anesthesia (sedative analgesic 3 kinds mixed drug: see below)
Drug names

- Medetomidine hydrochloride: Domitor (1 mg/mL)
- Midazolam (10 mg/mL)
- Butorphanol tartrate: Vetorphale (5 mg/mL)
- Xylazine
- Sterile PBS
- Atipamezole hydrochloride: Antisedan (5 mg/mL)

(5) Terumo Surflow indwelling needle for peroral intratracheal intubation (SR-OT2225C 22G 1")
(6) Sterile phosphate buffer physiological saline (Phosphate-buffered saline: PBS) «4. Protocol of animal experiment»

[Anesthesia]

(1) Domitor (1.875 mg/1.875 mL), Midazolam (20 mg/2 mL), Vetorphale (12.5 mg/2.5 mL), xylazine (25 mg powder), and PBS (18.625 mL) were mixed together and adjusted to a total of 25 mL.
(2) The liquid prepared in the above (1) was sterilized with a 0.22 μm filter. The sterilized liquid was intraperitoneally administered to mice by 100 μL per 10 g of body weight.

[Creation of animal models]

In the present test example, the treatment groups were verified in terms of preventive treatment.

-Day -2: Administration day of pharmaceutical composition (i)-

1) Under general anesthesia, peroral intratracheal intubation was performed using a stand for intratracheal intubation.
2) To (1) Control group and (3) Non-treatment group, 50 pL of PBS was intratracheally infused.

To (2) Treatment group (the transairway administration group), 50 μL of a solution of the pharmaceutical composition (i), which had been prepared in the following manner immediately before administration, was intratracheally infused. Also, to (2) Treatment group (the transabdominal administration group), 50 μL of a solution of the pharmaceutical composition (i) for administration at a high concentration (120 pg/mouse), which had been prepared immediately before in the following manner, was intratracheally infused.

- 240 mg of the pharmaceutical composition (i) was dissolved in 1,000 pL of PBS (this corresponding to (nintedanib 120 pg/PBS 50 μL)). This was used for administration at a high concentration (120 pg/mouse), and part thereof was 2-fold diluted for administration at a moderate concentration (60 pg/mouse) and was further diluted for administration at a low concentration (20 pg/mouse).

3) At the end of the experiment, Antisedan solution (a total of 10 mL of Antisedan 0.15 mL (0.75 mg/0.15 mL) and PBS 9.85 mL) was intraperitoneally administered to mice by 100 pL per 10 g of body weight. The mice were observed until emergence while confirming their respiratory status on a moisture-retaining pad.

-Day 0: Administration day of bleomycin-

1) Under general anesthesia, peroral intratracheal intubation was performed using a stand for intratracheal intubation.
2) To (1) Control group, 50 pL of PBS was intratracheally infused.

To (2) Treatment group and (3) Non-treatment group, 50 pL of a bleomycin solution, which had been prepared in the following manner, was intratracheally infused.

- The amount of bleomycin administered was 1 to 3 U/1 kg of body weight. Regarding the average body weight of mice as 20 g, the bleomycin solution was finally adjusted with physiological saline to be from 20 to 60 mU/50 μL before administration. In the present test, administration was performed with 3 U/1 kg of body weight = 60 mU/50 pL.

3) At the end of the experiment, Antisedan solution (the formulation of which is described in the section "Day -2") was intraperitoneally administered to mice by 100 μL per 10 g of body weight. The mice were observed until emergence while confirming their respiratory status on a moisture-retaining pad.

-Day 21: Measurement day of respiratory function-

Under general anesthesia, the neck of the mice was incised to expose the trachea, followed by microincision. A Flexivent catheter was inserted into the trachea. The distal portion of the inserted site was ligated with a floss. The mice were connected to Flexivent to measure respiratory functions.

<Results>

FIG. 12 presents results obtained by studying change in body weight of mice in the Control group (the physiological saline administration group), the Treatment groups (the transairway treatment group and the transabdominal treatment group: in each of the groups, the amount of the pharmaceutical composition (i) administered was 120 pg as the amount of nintedanib), and the Non-treatment group (bleomycin pulmonary fibrosis model).

The change in body weight is believed to reflect disease progression especially in inflammatory disease models and is known to be an important parameter for predicting therapeutic effects.

As presented in FIG. 12, temporal reduction in body weight by the transairway administration of bleomycin under general anesthesia was observed in all of the groups. In the bleomycin pulmonary fibrosis model (Non-treatment group), procrastination in body weight reduction was observed, and the body weight was returned to the baseline after two weeks or longer had passed after the administration. In the transairway treatment group, the body weight change was not so different from that of the Control group and the body weight reduction was significantly suppressed as compared with the Non-treatment group. In the transabdominal treatment groups, the body weight reduction was not suppressed as compared with the transairway treatment group.

As to statistical analysis, a test of significant difference by one-way analysis of variance was followed by the Student-Newman-Keuls analysis. The presence of a significant difference was determined at a significance level of 5%.

FIG. 13A to FIG. 13D each present results obtained by verifying efficacy when the dose of nintedanib was varied.

As presented in FIG. 13A, in the bleomycin pulmonary fibrosis model (Non-treatment group: a nintedanib dose of 0 pg), a clear reduction in the maximal inspiratory capacity was observed, which reflected reduction in the lung volume. In the transairway treatment groups, reduction in the maximal inspiratory capacity was significantly suppressed in the groups that received 60 pg and 120 pg (as the amount of nintedanib) as compared with the Non-treatment group.

As presented in FIG. 13B, in the bleomycin pulmonary fibrosis model (Non-treatment group: a nintedanib dose of 0 pg), a clear reduction in the lung thorax compliance was observed, which reflected reduction in compliance of the lungs (including the thorax). In the transairway treatment groups, reduction in the lung thorax compliance was significantly suppressed in the group that received 120 pg (as the amount of nintedanib) as compared with the Non-treatment group.

As presented in FIG. 13C, in the bleomycin pulmonary fibrosis model (Non-treatment group: a nintedanib dose of 0 pg), a clear increase in the lung tissue elastance was observed, which reflected increase in the elastance of the lungs. In the transairway treatment groups, improvement in the lung tissue elastance was observed in a dose-dependent manner, and the increase in the lung tissue elastance was significantly suppressed in the group that received 120 pg (as the amount of nintedanib) as compared with the Non-treatment group.

As presented in FIG. 13D, in the bleomycin pulmonary fibrosis model (Non-treatment group: a nintedanib dose of 0 pg), a clear reduction in static lung compliance was observed, which reflected reduction in compliance of the lungs. In the transairway treatment groups, improvement in the static lung compliance was observed in a dose-dependent manner, and the reduction in the static lung compliance was significantly suppressed in the group that received 60 pg or 120 pg (as the amount of nintedanib) as compared with the Non-treatment group.

As to statistical analysis, a test of significant difference by the Kruscal-Wallis test was followed by the Bonferroni post hoc analysis. The presence of a significant difference was determined at a significance level of 5%.

FIG. 14A to FIG. 14D each present results obtained by verifying efficacy when the route of administration of nintedanib was varied. In both the transairway treatment groups and the transabdominal treatment groups, the dose of the pharmaceutical composition (i) was 120 pg as the amount of nintedanib.

As presented in FIG. 14A, in the transairway treatment groups, reduction in the maximum respiratory capacity was significantly suppressed as compared with the Non-treatment group. In the transabdominal treatment groups, the reduction in the maximum respiratory capacity was not significantly suppressed as compared with the Non-treatment group.

As presented in FIG. 14B, in the transairway treatment groups, reduction in the lung thorax compliance was significantly suppressed as compared with the Non-treatment group. In the transabdominal treatment groups, the reduction in the lung thorax compliance was not significantly suppressed as compared with the Non-treatment group.

As presented in FIG. 14C, in the transairway treatment groups, increase in the lung tissue elastance was significantly suppressed as compared with the Non-treatment group. In the transabdominal treatment groups, the increase in the lung tissue elastance was not significantly suppressed as compared with the Non-treatment group.

As presented in FIG. 14D, in the transairway treatment groups, reduction in the static lung compliance was significantly suppressed as compared with the Non-treatment group. In the transabdominal treatment groups, the reduction in the static lung compliance was not significantly suppressed as compared with the Non-treatment group.

As to statistical analysis, a test of significant difference by one-way analysis of variance was followed by the Bonferroni analysis. The presence of a significant difference was determined at a significance level of 5%.

As presented above, the pharmaceutical composition of the present invention allows a poorly water-soluble pharmaceutical compound to rapidly dissolve in a state of having its physiological activity.

Hitherto, the route of administration of nintedanib is limited to oral administration, and the dose thereof has been set to be relatively high due to its low bioavailability. In many cases, tolerability cannot be acquired due to adverse events such as symptoms of digestive organs, and the dose is forced to decrease or discontinuation is unavoidable. It is however found that the pharmaceutical composition of the present invention enables transairway administration, and various pulmonary function parameters seen in the bleomycin pulmonary fibrosis model are significantly improved at a dose 1/10 the dose for oral administration. According to the pharmaceutical composition of the present invention, therefore, it is possible to provide a new route of administration of a poorly water-soluble pharmaceutical compound that has had difficulty in being administered via any other routes than oral administration.

Aspects of the present invention are as follows, for example.

<1> A pharmaceutical composition including

particles each containing a water-soluble base material and a poorly water-soluble compound,
the water-soluble base material containing a rapidly water-soluble compound,
wherein the poorly water-soluble compound is a kinase inhibitor and exists in an amorphous state in the water-soluble base material.

<2> The pharmaceutical composition according to <1> above, wherein the pharmaceutical composition can be prepared at time of use.
<3> The pharmaceutical composition according to <1> or <2> above, wherein the rapidly water-soluble compound is at least one selected from the group consisting of monosaccharides and disaccharides.
<4> The pharmaceutical composition according to any one of <1> to <3> above, wherein at time of administration, the pharmaceutical composition is prepared at time of use by being dissolved in water or physiological saline.

<5> The pharmaceutical composition according to any one of <1> to <4> above, wherein a solution of the pharmaceutical composition dissolved in water or physiological saline is atomized and administered.

<6> The pharmaceutical composition according to any one of <1> to <5> above, wherein a route of administration is a topical administration.

<7> The pharmaceutical composition according to any one of <1> to <6> above, wherein the pharmaceutical composition is used for a disease caused by activation of a kinase.

<8> A functional particle including:

a water-soluble base material; and
a poorly water-soluble compound,
the water-soluble base material containing a rapidly water-soluble compound,
wherein the poorly water-soluble compound exists in an amorphous state in the water-soluble base material.

<9> The functional particle according to <8> above, wherein the rapidly water-soluble compound is at least one selected from the group consisting of monosaccharides and disaccharides.

<10> The functional particle according to <8> or <9> above, wherein an amount of the poorly water-soluble compound is 75% by mass or less.

<11> The functional particle according to any one of <8> to <10> above, wherein an amount of the poorly water-soluble compound is 10% by mass or more but 50% by mass or less.

<12> The functional particle according to any one of <9> to <11> above, wherein a volume average particle diameter (Dv) of the functional particle is 0.5 μm or more but 50 μm or less.

<13> The functional particle according to any one of <8> to <12> above, wherein a volume average particle diameter (Dv) of the functional particle is 0.5 μm or more but 20 μm or less.

<14> A method for producing functional particles, the method including:

discharging a liquid containing a rapidly water-soluble compound and a poorly water-soluble compound from a discharging hole to form liquid droplets; and
solidifying the liquid droplets to form particles.

<15> The method according to <14> above,
wherein the discharging includes applying vibration to a composition housed in a liquid-column-resonance liquid chamber to form standing waves through liquid column resonance and discharging the composition from the discharging hole, the discharging hole being formed in an amplification direction of the standing waves and in regions that correspond to anti-nodes of the standing waves.

The pharmaceutical composition according to any one of <1> to <7> above can solve the existing problems in the art and can achieve the object of the present invention.

The functional particle according to any one of <8> to <13> above and the method according to <14> or <15> above can solve the existing problems in the art and allow the poorly water-soluble compound to rapidly dissolve.

Reference Signs List

| | |
|---|---|
| 1 | apparatus for producing functional particles |
| 11 | liquid droplet forming unit |
| 12, 101 | conveyance gas flow |
| 14 | liquid |
| 19 | discharging hole |
| 21 | liquid droplets |

## Claims

**1.** A pharmaceutical composition comprising

particles each containing a water-soluble base material and a poorly water-soluble compound,

the water-soluble base material containing a rapidly water-soluble compound, wherein the poorly water-soluble compound is a kinase inhibitor and exists in an amorphous state in the water-soluble base material.

**2.** The pharmaceutical composition according to claim 1, wherein the pharmaceutical composition can be prepared at time of use.

**3.** The pharmaceutical composition according to claim 1 or 2, wherein the rapidly water-soluble compound is at least one selected from the group consisting of monosaccharides and disaccharides.

**4.** The pharmaceutical composition according to any one of claims 1 to 3, wherein at time of administration, the pharmaceutical composition is prepared at time of use by being dissolved in water or physiological saline.

**5.** The pharmaceutical composition according to any one of claims 1 to 4, wherein a solution of the pharmaceutical composition dissolved in water or physiological saline is atomized and administered.

**6.** The pharmaceutical composition according to any one of claims 1 to 5, wherein a route of administration is a topical administration.

**7.** The pharmaceutical composition according to any one of claims 1 to 6, wherein the pharmaceutical composition is used for a disease caused by activation of a kinase.

FIG. 1

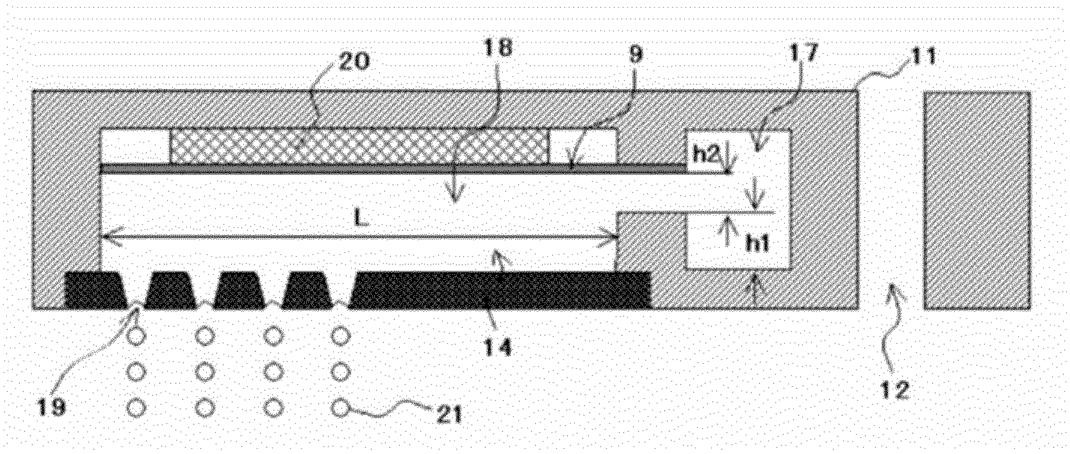


FIG. 2

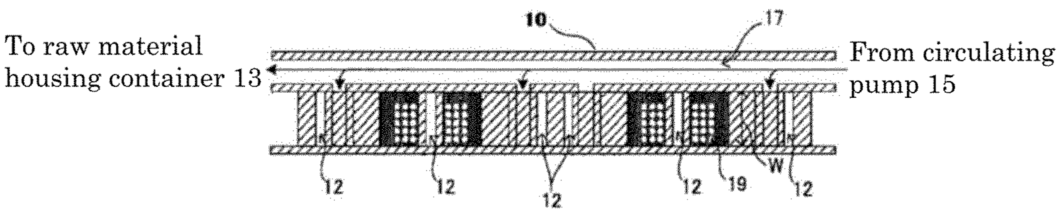


FIG. 3A

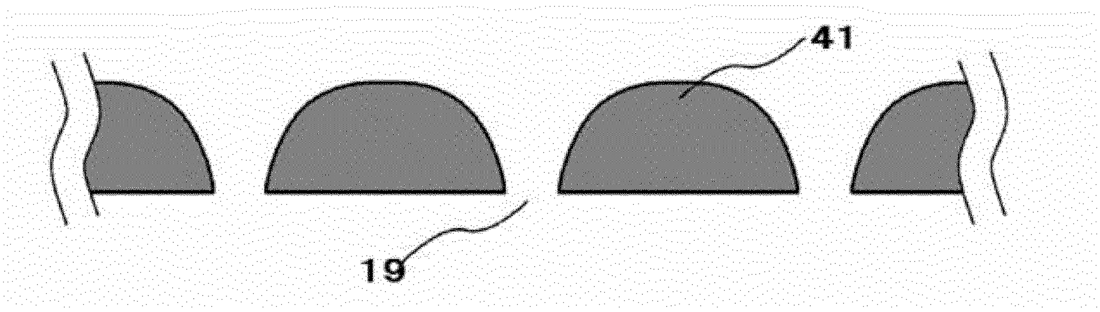

FIG. 3B

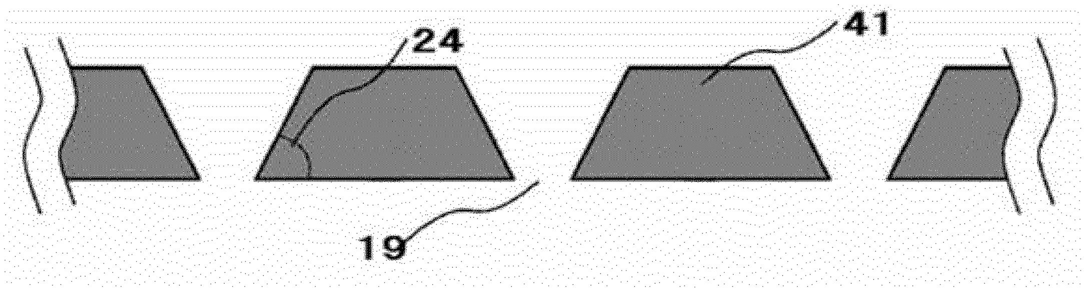


FIG. 3C

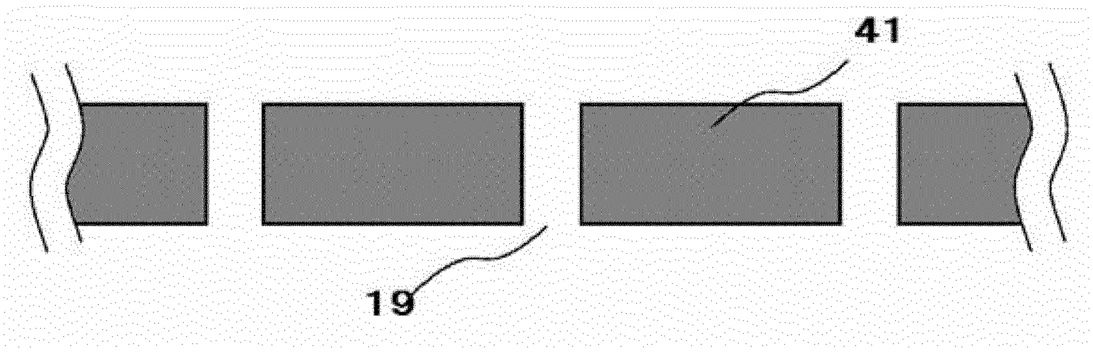


FIG. 3D

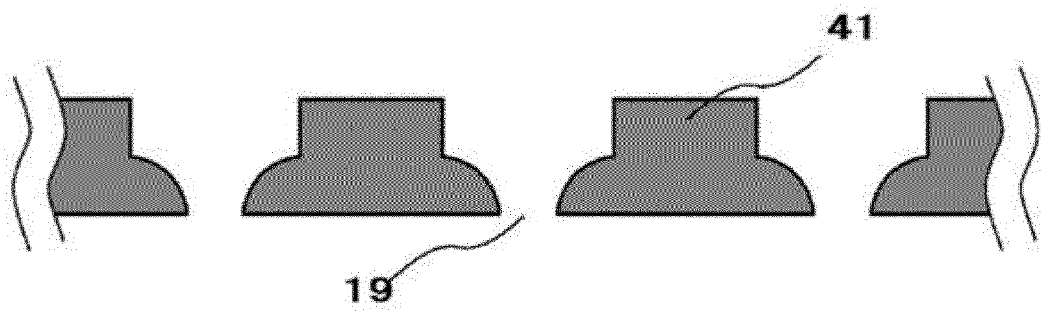

FIG. 4A

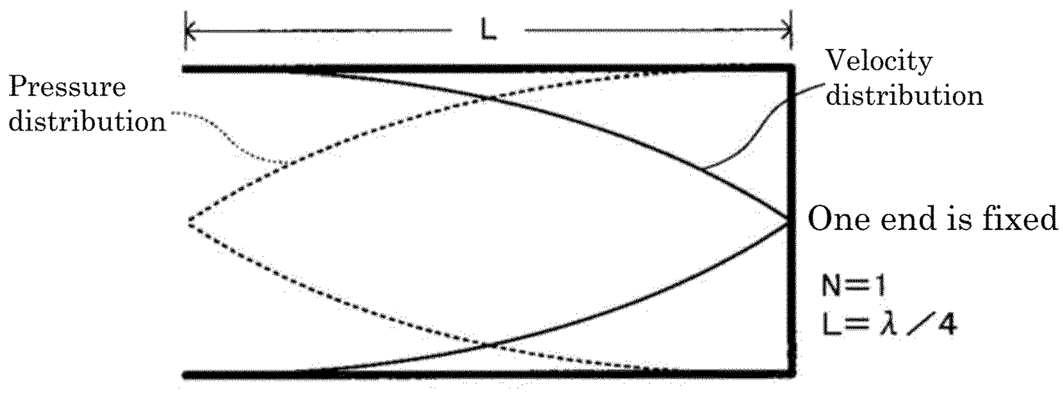
L
Pressure distribution
Velocity distribution
One end is fixed
N=1
L=λ/4

FIG. 4B

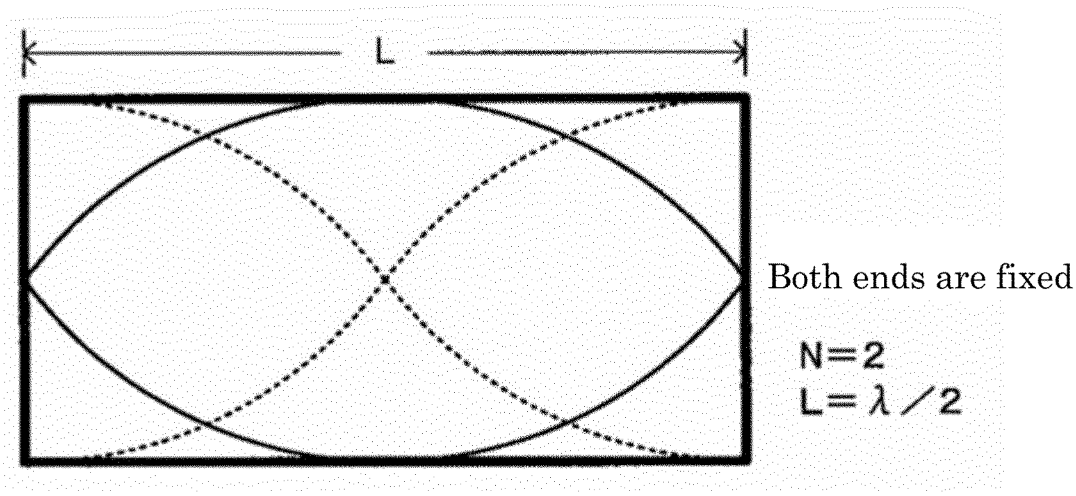
L
Both ends are fixed
N=2
L=λ/2

FIG. 4C

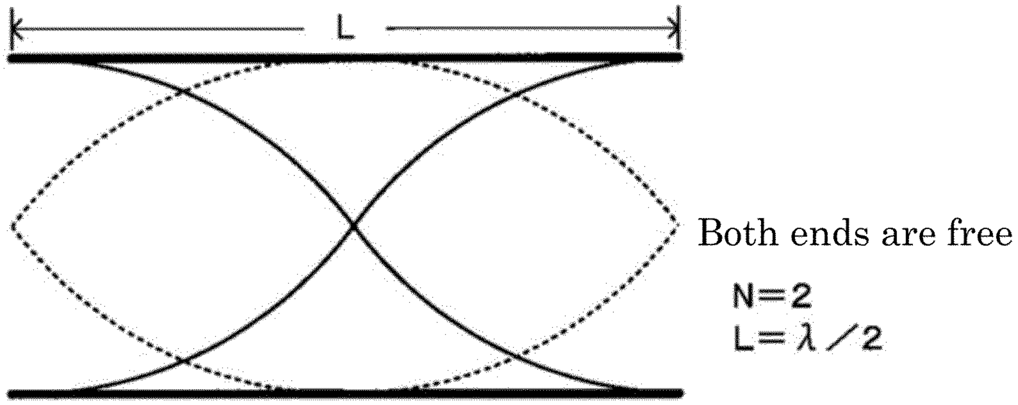


FIG. 4D

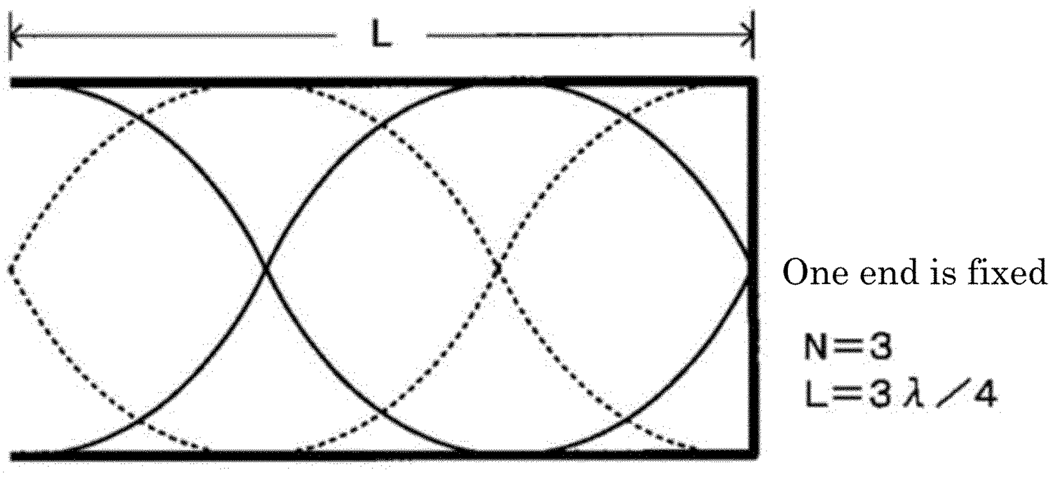

FIG. 5A

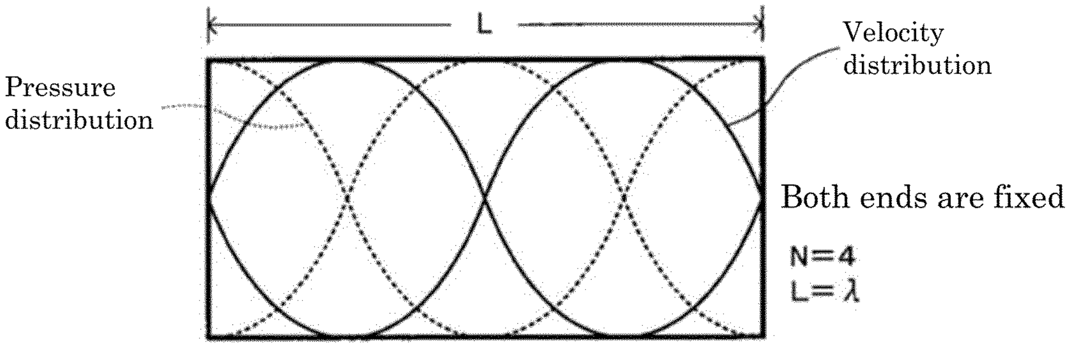


FIG. 5B

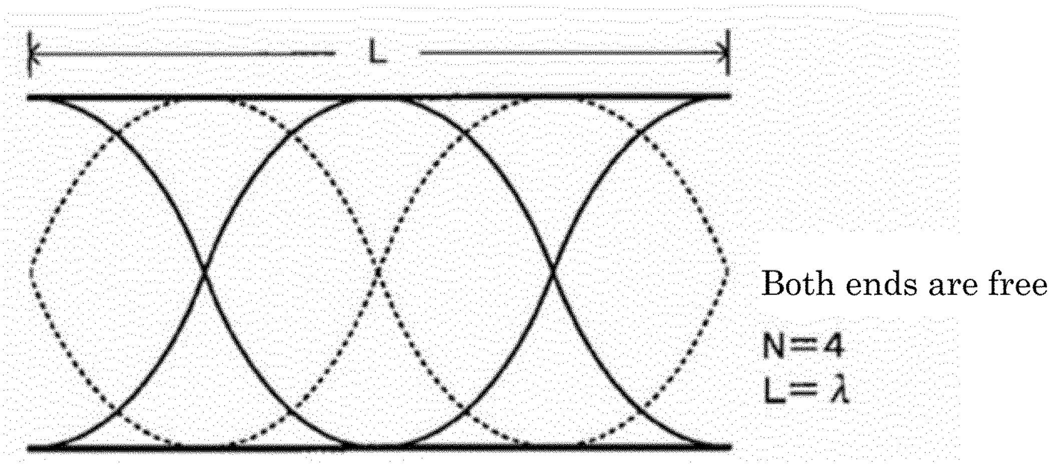


FIG. 5C

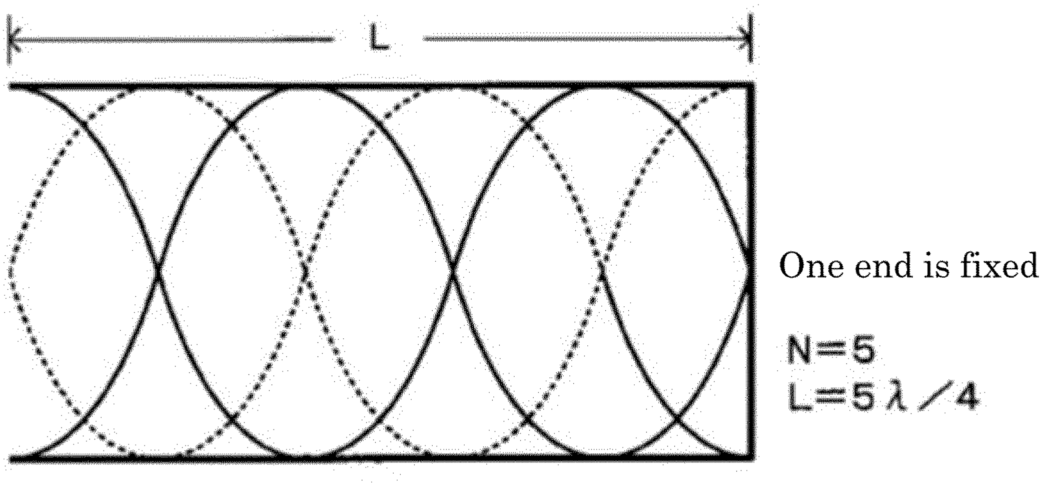

FIG. 6A

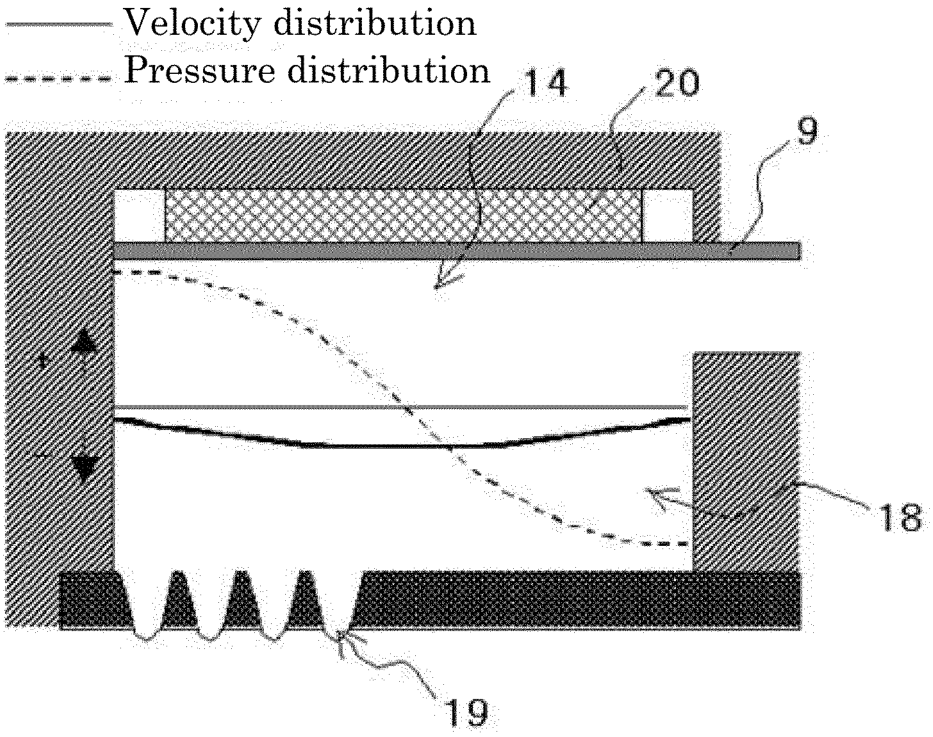


FIG. 6B

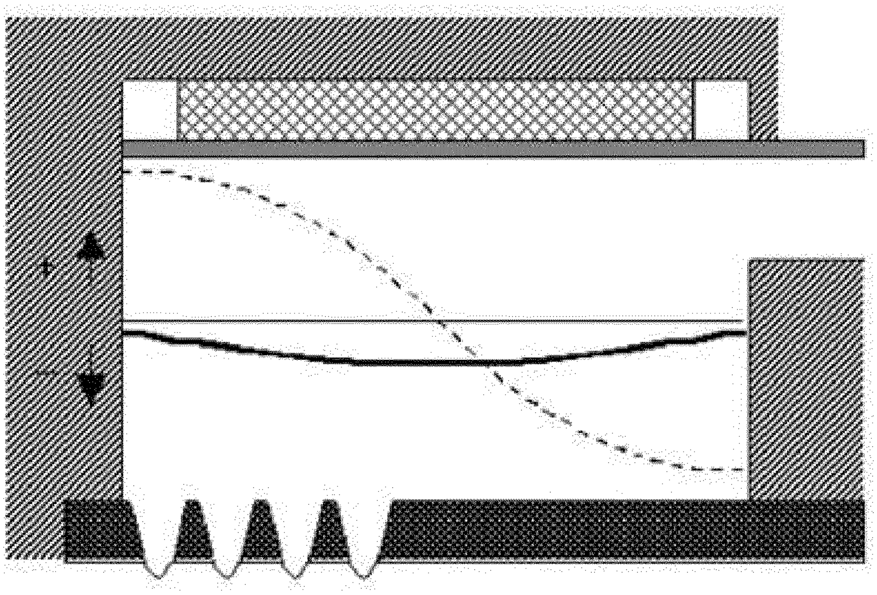

FIG. 6C

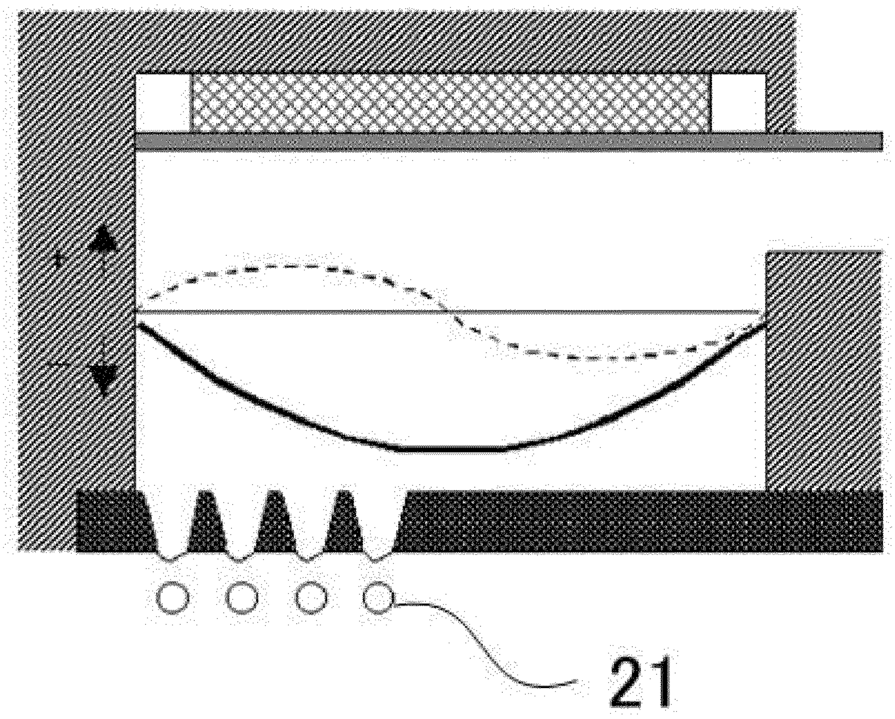


FIG. 6D

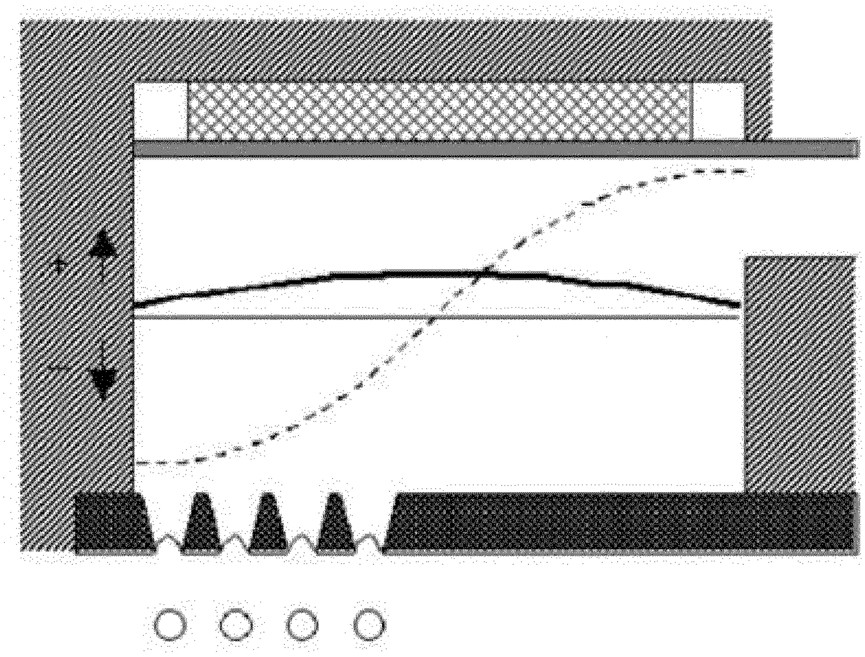

FIG. 6E

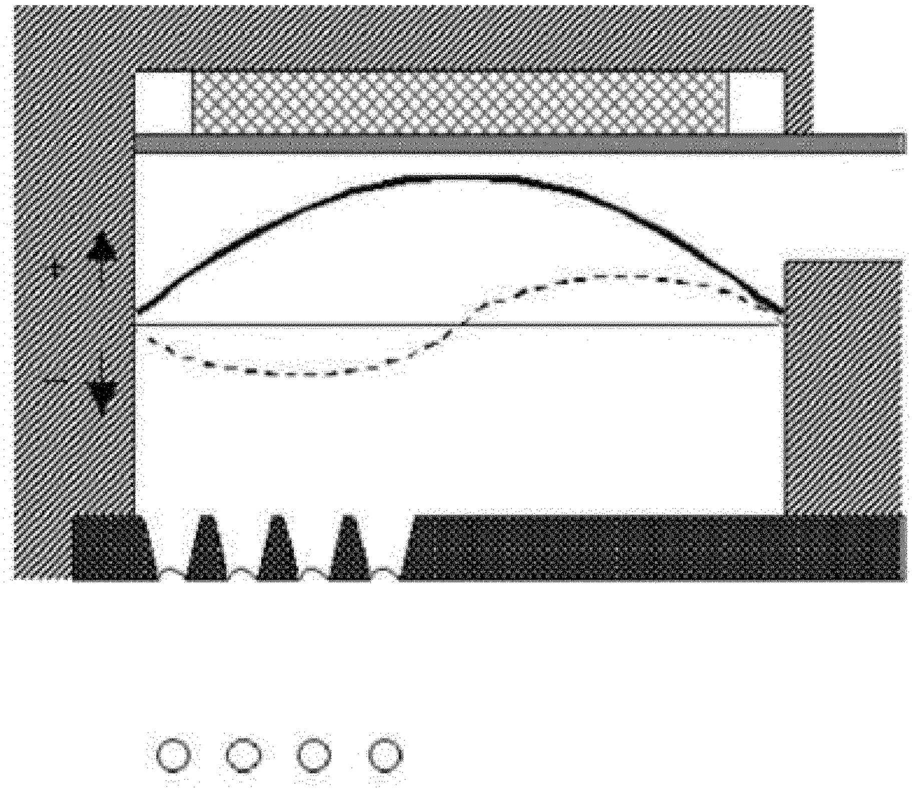

FIG. 7

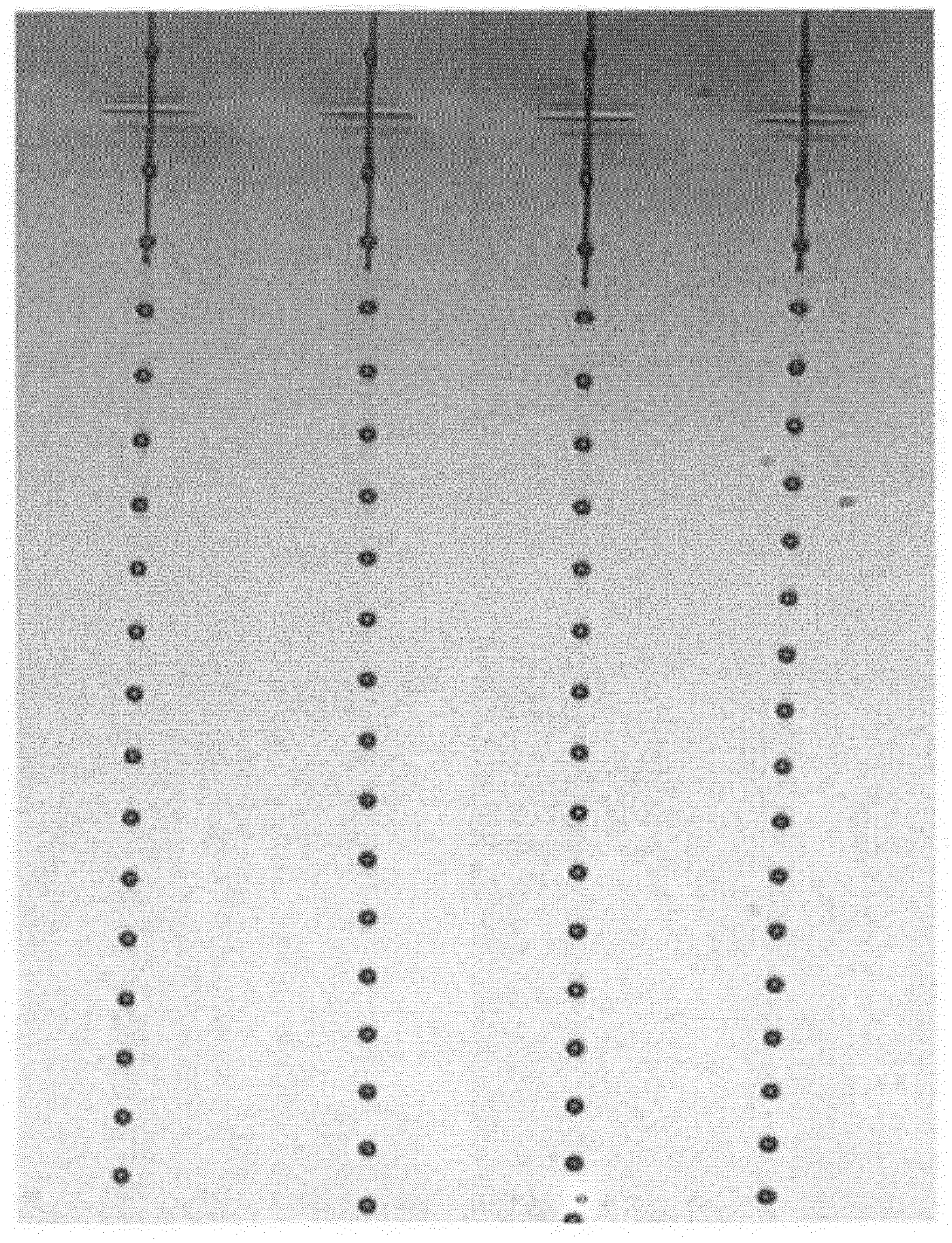

FIG. 8

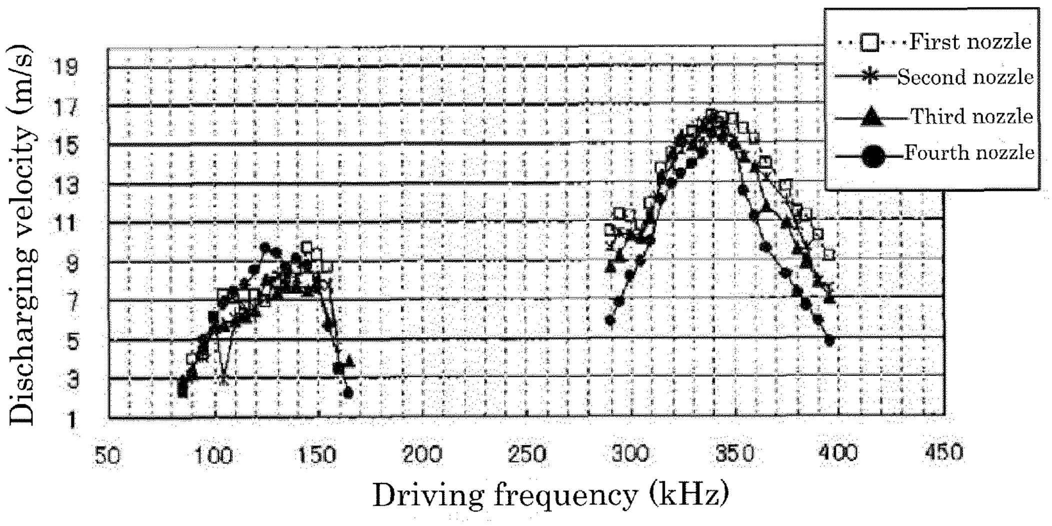
Discharging velocity (m/s)
Driving frequency (kHz)
First nozzle
Second nozzle
Third nozzle
Fourth nozzle
1
3
5
7
9
11
13
15
17
19
50
100
150
200
250
300
350
400
450

FIG. 9

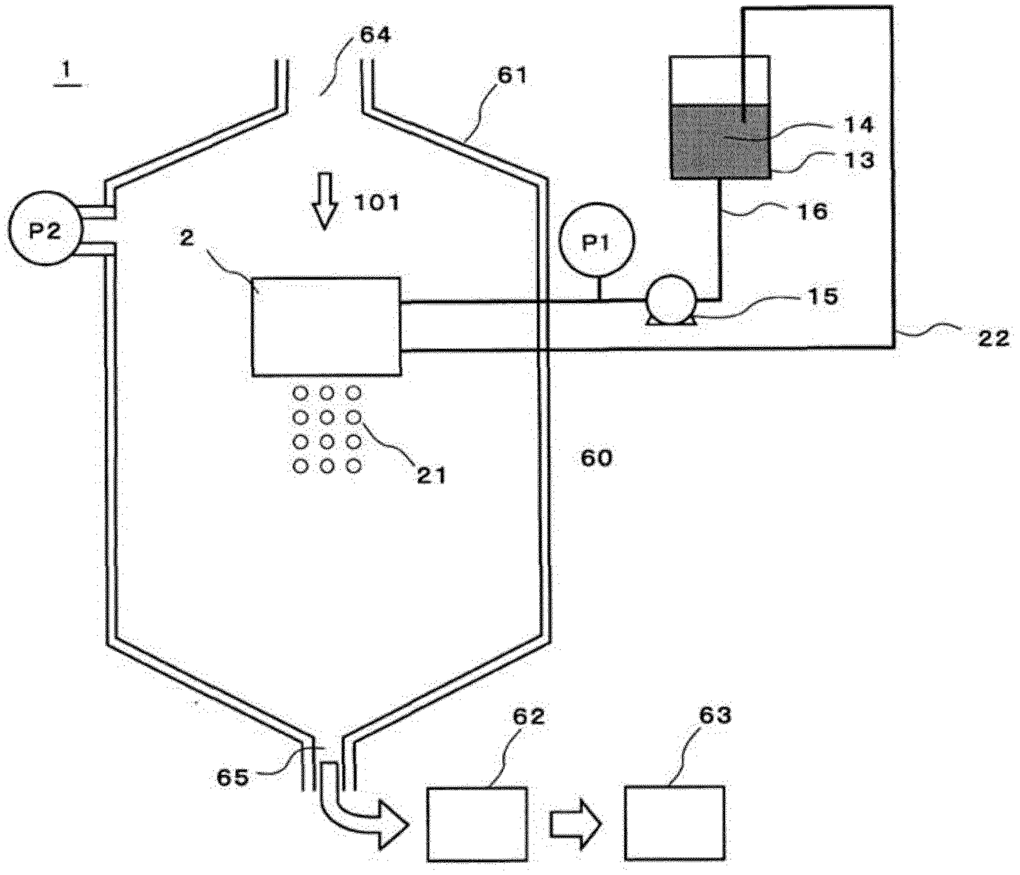
1
64
61
14
13
16
P2
101
2
P1
15
22
21
60
65
62
63

FIG. 10

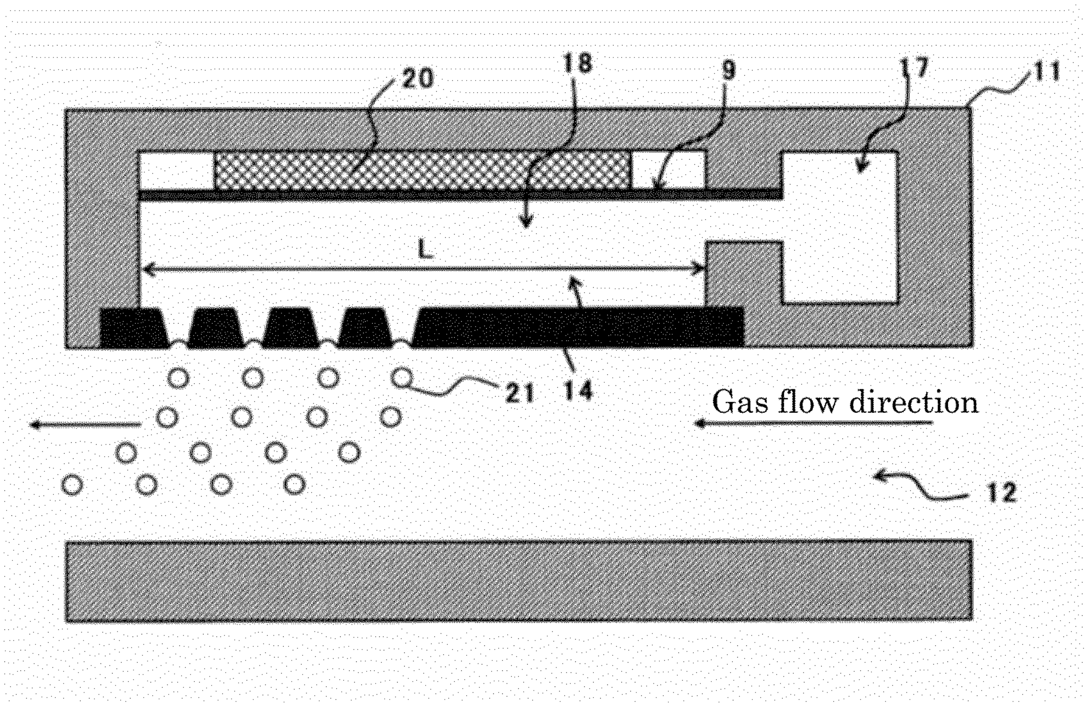
20
18
9
17
11
L
21
14
Gas flow direction
12

FIG. 11

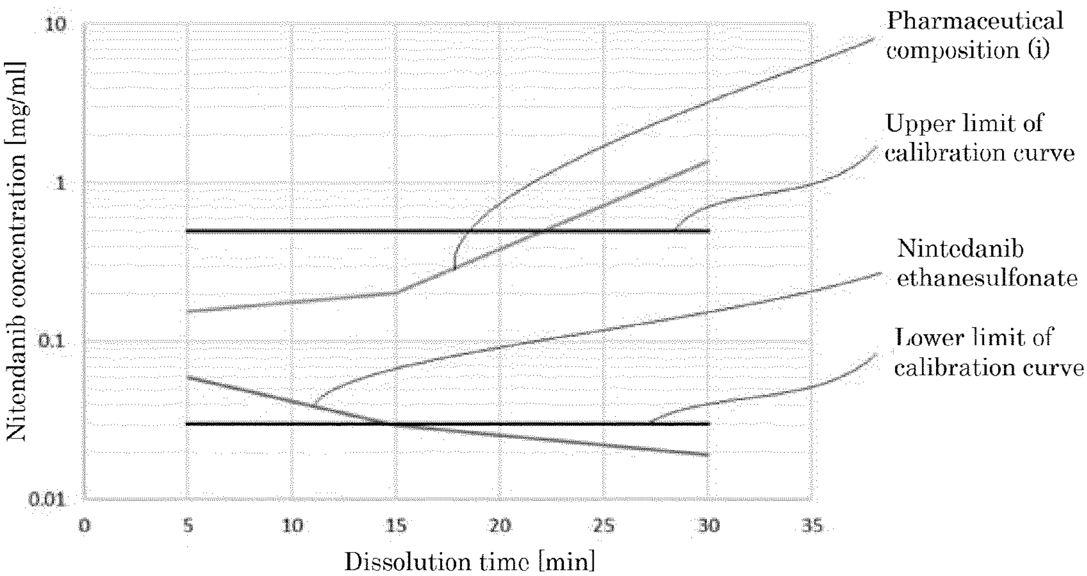
Nitendanib concentration [mg/ml]
10
1
0.1
0.01
0
5
10
15
20
25
30
35
Dissolution time [min]
Pharmaceutical composition (i)
Upper limit of calibration curve
Nintedanib ethanesulfonate
Lower limit of calibration curve

FIG. 12

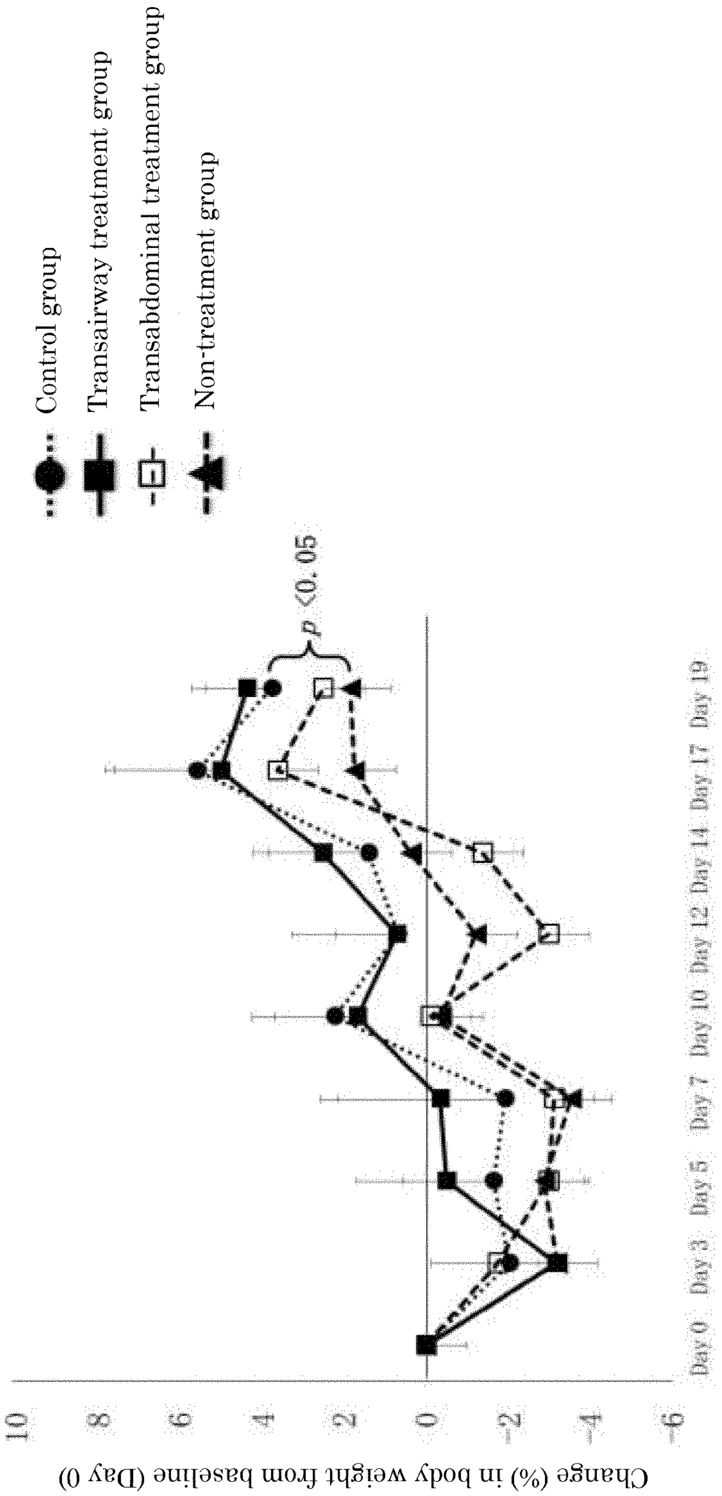
Control group
Transairway treatment group
Transabdominal treatment group
Non-treatment group
p <0.05
Change (%) in body weight from baseline (Day 0)
10
8
6
4
2
0
-2
-4
-6
Day 0
Day 3
Day 5
Day 7
Day 10
Day 12
Day 14
Day 17
Day 19

FIG. 13A

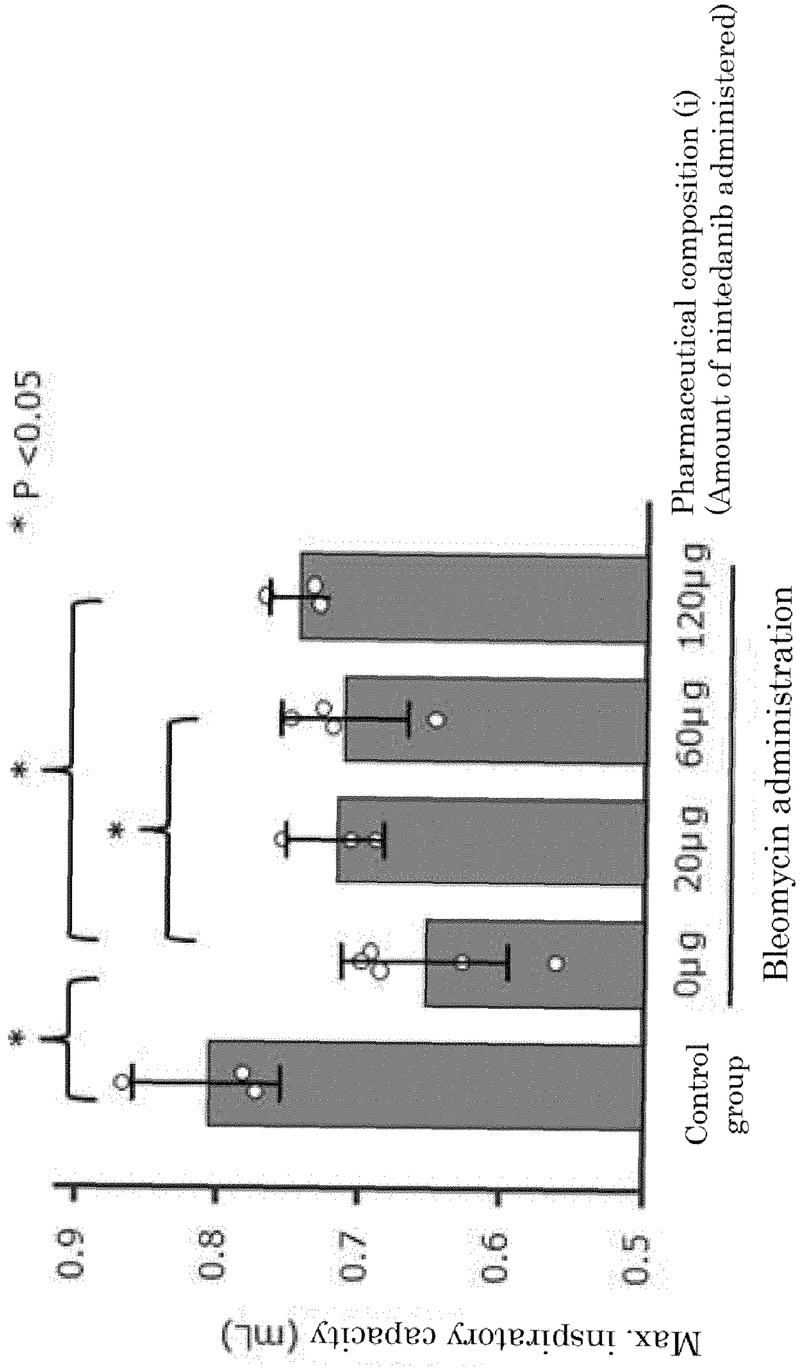
* P <0.05
Max. inspiratory capacity (mL)
0.9
0.8
0.7
0.6
0.5
*
*
*
Control group
0μg
20μg
60μg
120μg
Bleomycin administration
Pharmaceutical composition (i)
(Amount of nintedanib administered)

FIG. 13B

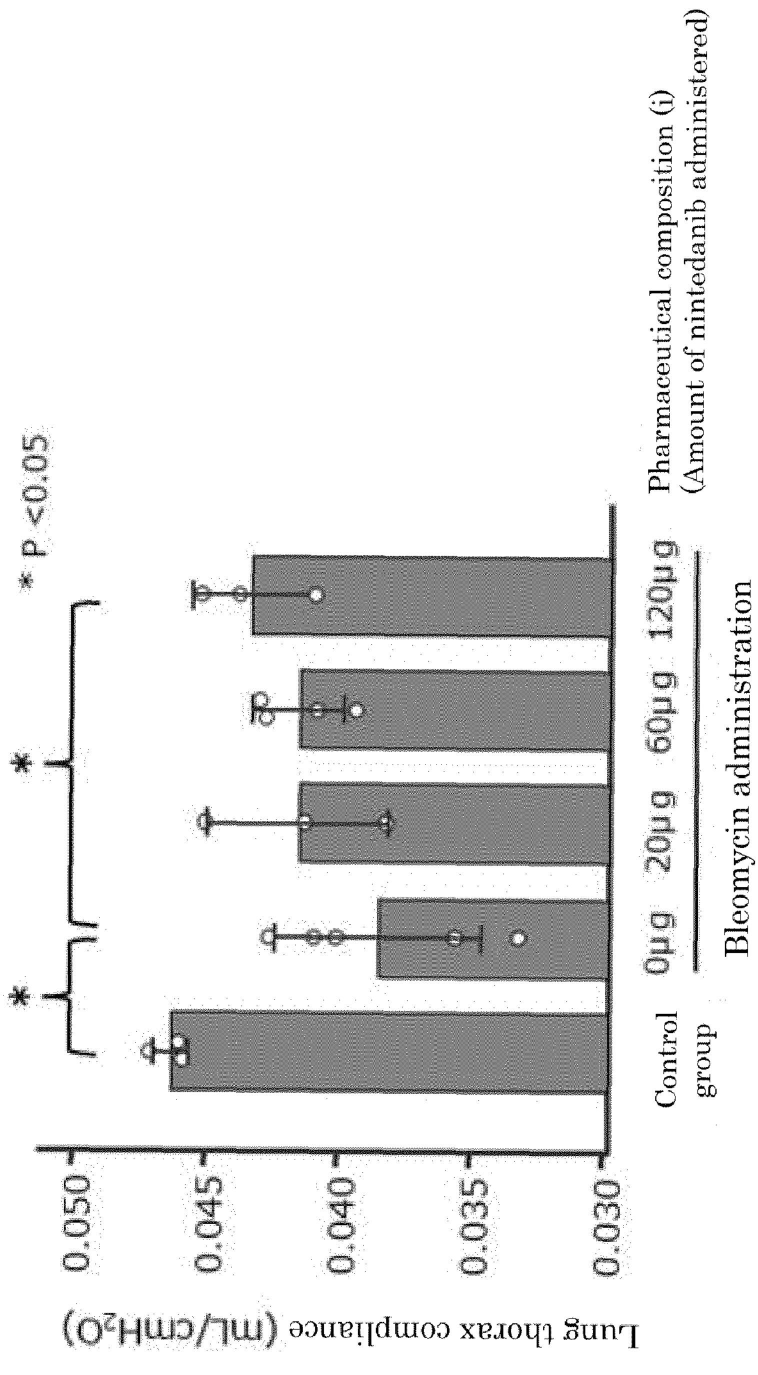
* P <0.05
*
*
Lung thorax compliance (mL/cmH₂O)
0.050
0.045
0.040
0.035
0.030
Control group
0μg
20μg
60μg
120μg
Bleomycin administration
Pharmaceutical composition (i)
(Amount of nintedanib administered)

FIG. 13C

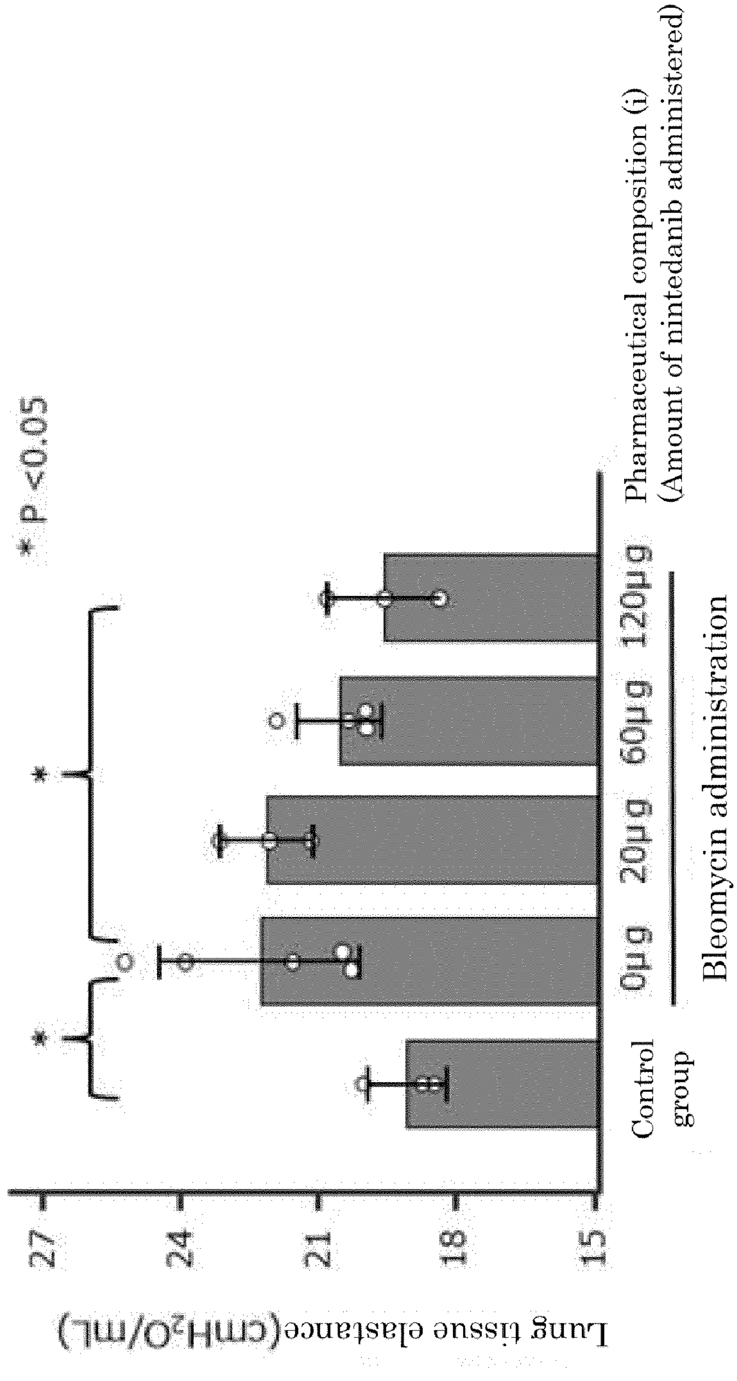
* P <0.05
*
*
Lung tissue elastance(cmH$_2$O/mL)
27
24
21
18
15
Control group
0μg
20μg
60μg
120μg
Pharmaceutical composition (i) (Amount of nintedanib administered)
Bleomycin administration

FIG. 13D

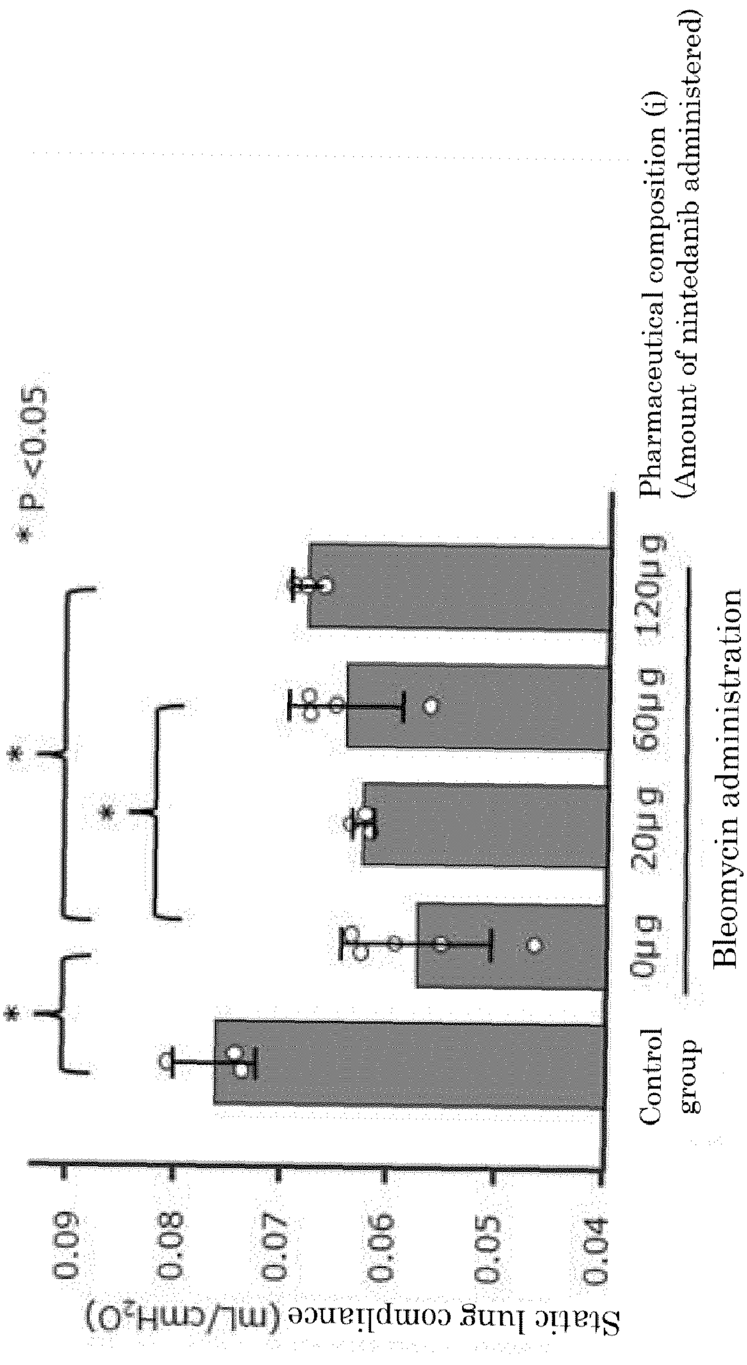
* P <0.05
*
*
*
Static lung compliance (mL/cmH$_2$O)
0.09
0.08
0.07
0.06
0.05
0.04
Control group
0μg
20μg
60μg
120μg
Pharmaceutical composition (i)
(Amount of nintedanib administered)
Bleomycin administration

FIG. 14A

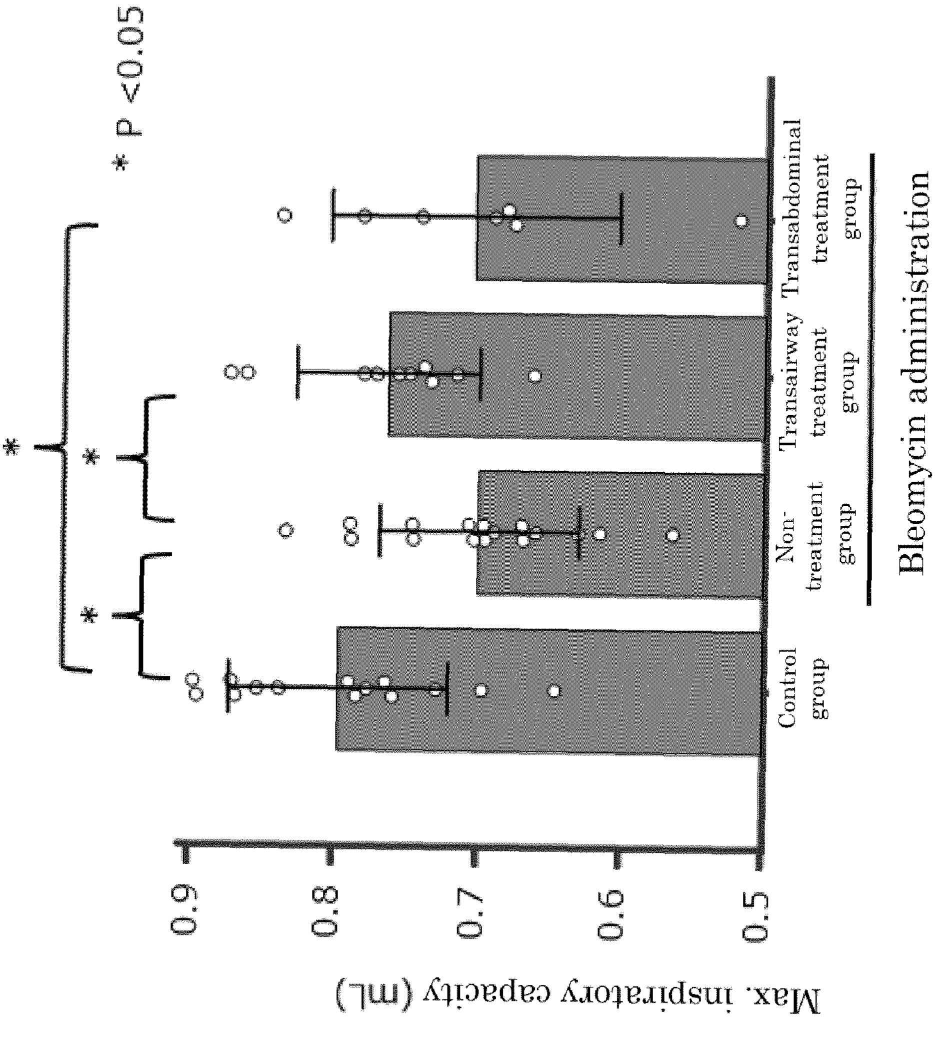
Max. inspiratory capacity (mL)
0.9
0.8
0.7
0.6
0.5
Control group
Non-treatment group
Transairway treatment group
Transabdominal treatment group
Bleomycin administration
*
* P <0.05

FIG. 14B

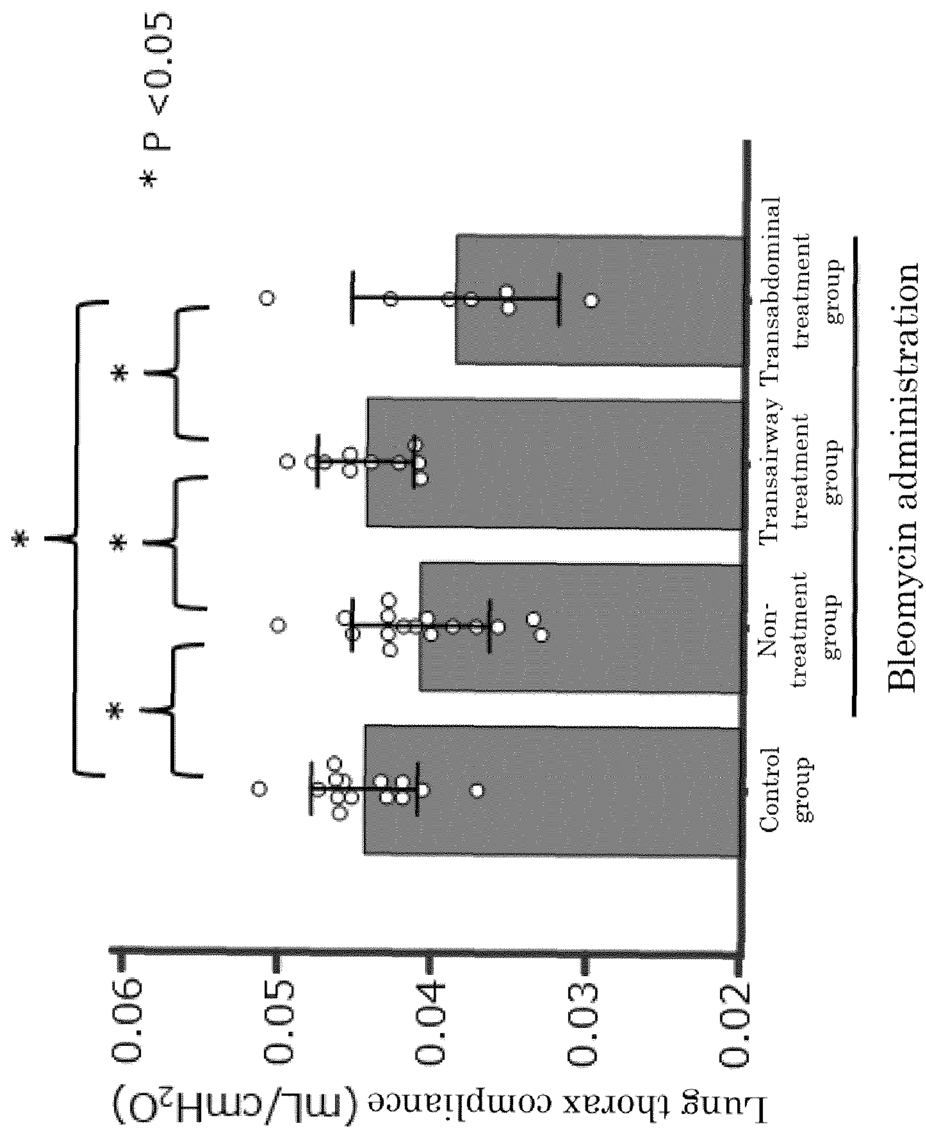
* P <0.05
Lung thorax compliance (mL/cmH₂O)
0.06
0.05
0.04
0.03
0.02
Control group
Non-treatment group
Transairway treatment group
Transabdominal treatment group
Bleomycin administration

FIG. 14C

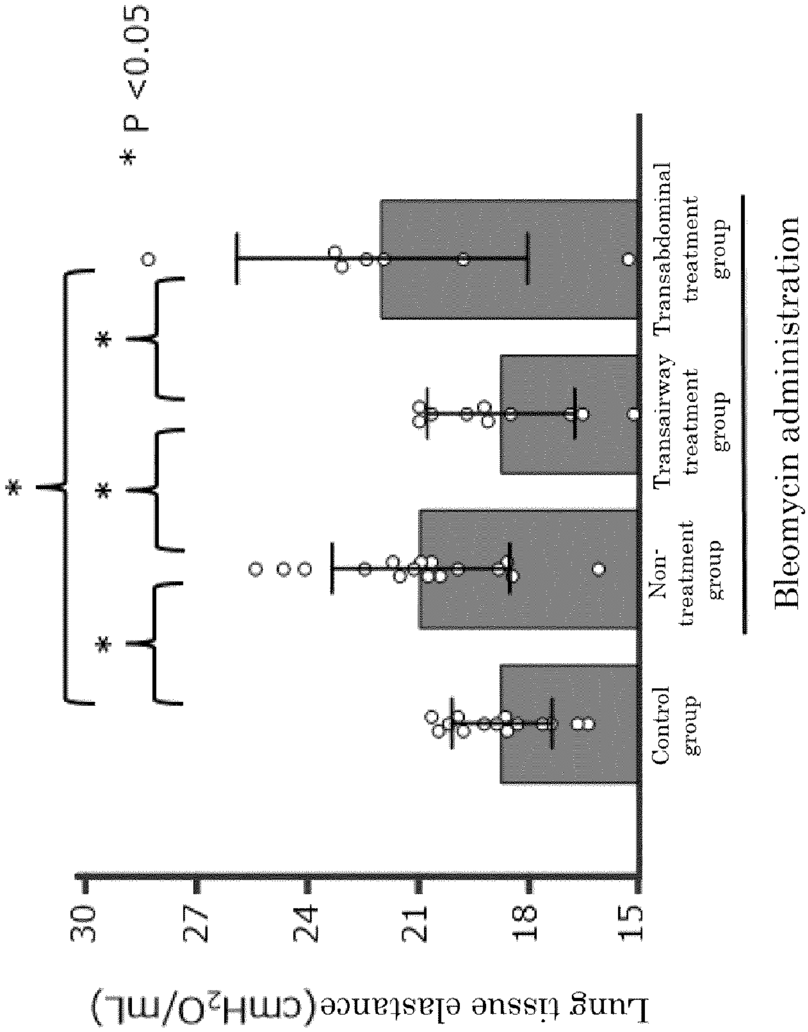
* P <0.05
*
*
*
*
Lung tissue elastance(cmH2O/mL)
30
27
24
21
18
15
Control group
Non-treatment group
Transairway treatment group
Transabdominal treatment group
Bleomycin administration

FIG. 14D

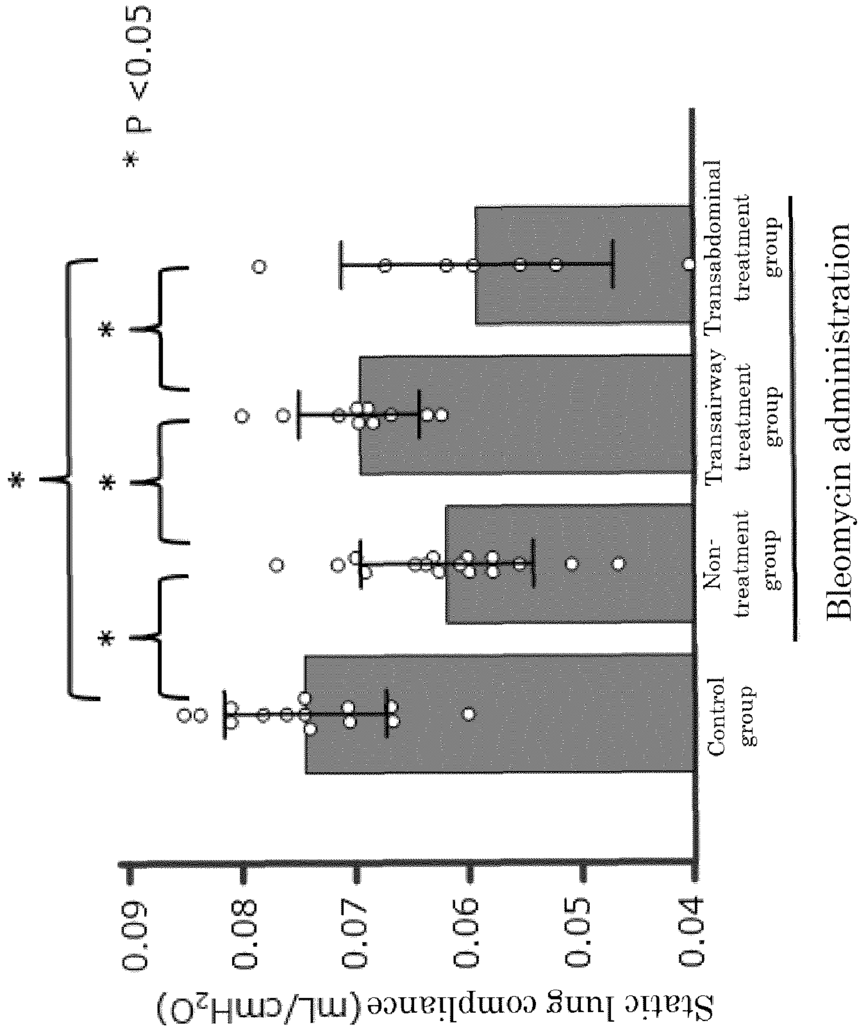
* P <0.05
Static lung compliance (mL/cmH2O)
0.09
0.08
0.07
0.06
0.05
0.04
Control group
Non-treatment group
Transairway treatment group
Transabdominal treatment group
Bleomycin administration
*
*
*
*

# INTERNATIONAL SEARCH REPORT

International application No.
PCT/JP2020/000711

A. CLASSIFICATION OF SUBJECT MATTER
Int.Cl. A61K45/00(2006.01)i, A61K9/12(2006.01)i, A61K9/14(2006.01)i, A61K47/26(2006.01)i
FI: A61K9/14, A61K9/12, A61K47/26, A61K45/00
According to International Patent Classification (IPC) or to both national classification and IPC

B. FIELDS SEARCHED
Minimum documentation searched (classification system followed by classification symbols)
Int.Cl. A61K45/00, A61K9/12, A61K9/14, A61K47/26

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII), CAplus/MEDLINE/EMBASE/BIOSIS/(STN)

C. DOCUMENTS CONSIDERED TO BE RELEVANT

| Category* | Citation of document, with indication, where appropriate, of the relevant passages | Relevant to claim No. |
|---|---|---|
| X | JP 11-509223 A (NOVARTIS AG) 17.08.1999 (1999-08-17), claims 1, 2, page 3, lines 9, 10, page 6, lines 26, 27, page 11, lines 14-16, page 14, lines 25-27, example 1 | 1-4, 7 |
| X | JP 2010-280685 A (ELAN PHARMA INTERNATL LTD.) 16.12.2010 (2010-12-16), claims 1-4, 6-8, examples 8, 9 | 1-3, 6-7 |
| X | JP 2017-31140 A (NIPPON KAYAKU CO., LTD.) 09.02.2017 (2017-02-09), claims 1-2, 7-9, paragraphs [0003], [0032], example 1 | 1-7 |
| A | JP 2010-515753 A (BOARD OF REGENTS UNIV OF TEXAS SYSTEM) 13.05.2010 (2010-05-13), claims 1-44 | 1-7 |
| A | JP 2017-160188 A (RICOH CO., LTD.) 14.09.2017 (2017-09-14), claims 1-5 | 1-7 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11.03.2020 | 24.03.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/000711

| | | |
|---|---|---|
| JP 11-509223 A | 17.08.1999 | WO 97/03654 A2<br>claims 1-2, page 1, line 10,<br>page 4, lines 21, 22,<br>page 9, line 26 to page 10,<br>line 1, page 14, lines 3-5,<br>example 1<br>EP 1281400 A2 |
| JP 2010-280685 A | 16.12.2010 | US 2003/0181411 A1<br>claims 1-4, 6-8,<br>exhibits 8, 9<br>WO 03/080024 A2 |
| JP 2017-31140 A | 09.02.2017 | US 2018/0214423 A1<br>claims 1-2, 7-9,<br>paragraphs [0002], [0003], [0138],<br>example A1<br>EP 3329916 A1 |
| JP 2010-515753 A | 13.05.2010 | WO 2008/127746 A1<br>claims 1-44 |
| JP 2017-160188 A | 14.09.2017 | WO 2017/150692 A1<br>claims 1-5 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2960169 B
- JP 5484910 B
- JP 2008292976 A
- JP 4647506 B
- JP 2010102195 A